# EUROPEAN PATENT SPECIFICATION

(11) **EP 3 743 206 B1**
(45) Date of publication and mention of the grant of the patent: **01.02.2023**
(21) Application number: 19706778.8
(22) Date of filing: 14.01.2019
(51) Int. Cl.: B01J 23/89, B01J 37/03, C07C 5/32, C07C 5/333

(54) **DEHYDROGENATION CATALYSTS AND METHODS FOR PREPARING AND USING THEM**
DEHYDRIERUNGSKATALYSATOREN UND VERFAHREN ZU IHRER HERSTELLUNG UND VERWENDUNG
CATALYSEURS DE DÉSHYDROGÉNATION, LEURS PROCÉDÉS DE PRÉPARATION ET D'UTILISATION

(30) Priority: 26.01.2018 US 201862622201 P
(43) Date of publication of application: 02.12.2020
(73) Proprietor: Clariant International Ltd, 4132 Muttenz (CH)
(72) Inventor: XING, Rong, Louisville, Kentucky 40241 (US); FRIDMAN, Vladimir, Louisville, Kentucky 40245 (US)
(74) Representative: Kuba, Stefan
(86) International application number: PCT/US2019/013438
(87) International publication number: WO 2019/147424

(56) References cited:
- EP-A2- 1 074 299
- CN-A- 105 582 929
- US-A1- 2017 120 222

## Description

### BACKGROUND OF THE DISCLOSURE

### Field of the Disclosure

This disclosure relates generally to catalyst materials and methods for preparing and using them. More particularly, the present disclosure relates to gallium-based catalysts comprising multiple metal oxide components, to methods for making such catalysts, and to methods for dehydrogenating hydrocarbons using such catalysts.

### Technical Background

Alkane dehydrogenation is a recognized process for production of a variety of useful hydrocarbon products, such as in the dehydrogenation of propane to make propene for use in the polymer industry, dehydrogenation of n-butane to produce n-butene or alkylate and butadiene useful in tire production, and the dehydrogenation of isobutane to make isobutylene suitable for conversion to methyl tert-butyl ether, isooctane, and alkylates to supplement and enrich gasolines. Current commercial catalysts useful for catalytic dehydrogenation of light alkanes include CrOx/Al₂O₃ and Pt-Sn/Al₂O₃ catalysts, which have been in use for decades.

CrOx/Al₂O₃ dehydrogenation catalysts typically contain a majority of their chromium in the Cr(lll) oxidation state on alumina surface. However, there typically remains a small amount of Cr(VI), which is carcinogenic and thus presents health risks during catalyst handling and operation. Pt-Sn/Al₂O₃ catalysts are expensive. Moreover, to provide a spent Pt-Sn/Al₂O₃ catalyst with initial activity, treatment during operation with Cl₂ containing gas is required. Such gases can be deadly and thus present significant risks during operation. They also can cause significant environmental chlorine pollution.

Gallium-based dehydrogenation catalysts have been known for about two decades. They are generally not hazardous, and their application presents no significant environmental issue. However, these catalysts have limitations in activity and stability, especially for the commercially important dehydrogenation of propane, n-butane, and isobutane.

US 2017/120222 A1, CN 105 582 929 A, and EP 1 074 299 A2 disclose gallium-containing dehydrogenation catalysts, their preparation process and use.

Accordingly, there remains a need for gallium-based dehydrogenation catalysts that provide improved activity and stability, especially in the dehydrogenation of propane and isobutane.

### SUMMARY OF THE DISCLOSURE

One aspect of the disclosure is a calcined dehydrogenation catalyst composition comprising
gallium oxide, present in the composition in an amount within the range of about 0.1 wt.% to about 30 wt.%, calculated as Ga₂O₃ on a calcined basis;
cerium oxide, present in the composition in an amount within the range of about 0.1 wt.% to about 15 wt.%, calculated as CeO₂ on a calcined basis;
a promoter M1 selected from platinum, iridium, lanthanum, or a mixture thereof, present in the composition in an amount within the range of about 0.005 wt.% to about 4 wt.%, calculated as oxide on a calcined basis;
a promoter M2 selected from the group 1 elements, present in the composition in an amount within the range of about 0.05 wt.% to about 3 wt.%, calculated as oxide on a calcined basis; and a promotor M3 selected from Ca, Sr, Ba, Fe, present in the composition in an amount within the range of 0.05 wt.% to 10 wt.%, calculated as oxide on a calcined basis, and
a support S1 selected from alumina, silica, zirconia, titania, or a mixture thereof, present in the composition in an amount within the range of about 60 wt.% to about 99 wt.%, calculated as oxide on a calcined basis.

Another aspect of the disclosure is a method for preparing the dehydrogenation catalyst composition of the invention, comprising providing an aqueous solution comprising a gallium source; a cerium source; an M1 source; an M2 source; an M3 source; and an S1 source; forming a solid from the solution; and calcining the composition so formed.

Another aspect is
the use of a catalyst composition of the invention for the dehydrogenation of a hydrocarbon.

### BRIEF DESCRIPTION OF THE DRAWINGS

Figure 1 is a bar graph showing (left-to-right in each set of bars) propane dehydrogenation conversion and propylene selectivity data for a variety of catalysts described herein.
Figure 2 is a line graph showing activity and selectivity data for a variety of catalysts described herein (top-to-bottom at the right side of the set of traces, comm., A3, A4, A5, A6 and A7, over three dehydrogenation cycles.
Figure 3 is a bar graph showing (left-to-right in each set of bars) coke yield and C₁-C₂ byproduct yield data of a propane dehydrogenation cycle using a variety of catalysts described herein.
Figure 4 is a bar graph showing (left-to-right in each set of bars) propane conversion and propylene selectivity data for a variety of catalysts described herein.
Figure 5 is a bar graph showing (left-to-right in each set of bars) propane conversion and propylene selectivity data for a variety of catalysts described herein.
Figure 6 is a bar graph showing (left-to-right in each set of bars) propane conversion and propylene selectivity data for a variety of catalysts described herein.

### DETAILED DESCRIPTION

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

The disclosure relates to dehydrogenation catalyst compositions that include gallium oxide, cerium oxide, one or more promoters selected from platinum, iridium, lanthanum, a mixture thereof, and the group 1 elements, Ca, Sr, Ba, Fe, and a mixed oxide support. The disclosure demonstrates that such catalysts, which may advantageously be free of chromium-containing materials, can exhibit performance comparable to or even better than conventional, commercially available catalysts.

One aspect of the disclosure is a calcined dehydrogenation catalyst composition. The catalyst composition includes gallium oxide, present in the composition in an amount within the range of 0.1 wt% to 30 wt% calculated as Ga₂O₃ on a calcined basis, and cerium oxide, present in the composition in an amount within the range of 0.1 wt% to 15 wt%, calculated as CeO₂ on a calcined basis. The catalyst composition includes a promoter, M1, selected from Pt, Ir, La, and a mixture thereof, present in the composition in an amount within the range of 0.005 wt% to 4 wt.%, and a promoter, M2, selected from group 1 elements (e.g., Li, Na, K, Cs), present in the composition in an amount within the range of 0.05 wt% to 3 wt%, each calculated as oxide on a calcined basis. The catalyst composition includes a promotor M3 selected from Ca, Sr, Ba, Fe, present in the composition in an amount within the range of 0.05 wt.% to 10 wt.%, calculated as oxide on a calcined basis. And the catalyst composition includes a support, S1, selected from alumina, silica, zirconia, titania, and a mixture thereof, present in the composition in an amount within the range of 60 wt% to 99 wt%, calculated as oxide on a calcined basis.

In certain embodiments as otherwise described herein, a catalyst composition includes a promoter, M1-B, selected from Sn and Pd, present in the composition in an amount up to about 0.5 wt% (e.g., within the range of 0.005 wt% to 0.5 wt.%), calculated as oxide on a calcined basis.

The catalyst composition includes a promoter, M3, selected from Ca, Sr, Ba, Fe present in the composition in an amount within the range of 0.05 wt.% to 10 wt%, calculated as oxide on a calcined basis.

As used herein, the term "oxide," including, e.g., "mixed oxide," "gallium oxide," "cerium oxide," etc., includes oxides in all forms and crystalline phases. For example, "gallium oxide" includes Ga₂O₃, Ga₂Oₓ wherein x is within the range of 1 to 3, etc. Unless otherwise indicated, regardless of the actual stoichiometry of the oxide, oxides are calculated as the most stable oxide for purposes of weight percent determinations. For example, the person of ordinary skill in the art will appreciate that a non-stoichiometric oxide of gallium, or even another form of gallium, may still be calculated as Ga₂O₃. Moreover, unless otherwise indicated, the compositions are described on an as-calcined basis.

Without intending to be bound by theory, the present inventors believe that the gallium oxide acts as a primary catalytic species in dehydrogenation reactions mediated by the catalyst compositions described herein. As described above, in one aspect of the compositions of the disclosure, gallium oxide is present in an amount within the range of 0.1 wt.% to 30 wt.%, calculated as Ga₂O₃ on a calcined basis. For example, in certain embodiments of the compositions as otherwise described herein, gallium oxide is present in an amount within the range of 0.1 wt.% to 27.5 wt.%, or 0.1 wt.% to 25 wt.%, or 0.1 wt.% to 22.5 wt.%, or 0.1 wt.% to 20 wt.%, or 0.1 wt.% to 17.5 wt.%, or 0.1 wt.% to 15 wt.%, or 0.1 wt.% to 12.5 wt.%, or 0.1 wt.% to 10 wt.%, or 0.5 wt.% to 30 wt.%, or 1 wt.% to 30 wt.%, or 2.5 wt.% to 30 wt.%, or 5 wt.% to 30 wt.%, or 7.5 wt.% to 30 wt.%, or 10 wt.% to 30 wt.%, or 12.5 wt.% to 30 wt.%, or 15 wt.% to 30 wt.%, or 17.5 wt.% to 30 wt.%, or 20 wt.% to 30 wt.%, or 0.5 wt.% to 27.5 wt.%, or 0.5 wt.% to 25 wt.%, or 1 wt.% to 22.5 wt.%, or 1 wt.% to 20 wt.%, or 1.5 wt.% to 17.5 wt.%, or 2 wt.% to 15 wt.%, calculated as Ga₂O₃ on a calcined basis.

Without intending to be bound by theory, the present inventors believe that the cerium oxide acts as a primary catalytic species in dehydrogenation reactions mediated by the catalyst compositions described herein. As described above, in one aspect of the compositions of the disclosure, cerium oxide is present in an amount within the range of 0.1 wt.% to 15 wt.%, calculated as CeO₂ on a calcined basis. For example, in certain embodiments of the compositions as otherwise described herein, cerium oxide is present in an amount within the range of 0.1 wt.% to 14 wt.%, or 0.1 wt.% to 13 wt.%, or 0.1 wt.% to 12 wt.%, or 0.1 wt.% to 11 wt.%, or 0.1 wt.% to 10 wt.%, or 0.1 wt.% to 9 wt.%, or 0.1 wt.% to 8 wt.%, or 0.1 wt.% to 7 wt.%, or 0.1 wt.% to 6 wt.%, or 0.1 wt.% to 5 wt.%, or 0.1 wt.% to 4 wt.%, or 0.1 wt.% to 3 wt.%, or 0.5 wt.% to 15 wt.%, or 1 wt.% to 15 wt.%, or 2 wt.% to 15 wt.%, or 3 wt.% to 15 wt.%, or 4 wt.% to 15 wt.%, or 5 wt.% to 15 wt.%, or 6 wt.% to 15 wt. %, or 7 wt.% to 15 wt.%, or 8 wt.% to 15 wt.%, or 9 wt.% to 15 wt.%, calculated as CeO2 on a calcined basis.

As described above, in one aspect of the compositions of the disclosure, M1, selected from platinum, iridium, lanthanum, or a mixture thereof, is present in an amount within the range of 0.005 wt.% to 4 wt.%, calculated as oxide on a calcined basis. For example, in certain embodiments of the compositions as otherwise described herein, M1 is selected from Pt and Ir. In another example, in certain embodiments of the compositions as otherwise described herein, M1 is selected from a mixture of Pt and La, and a mixture of Ir and La. In certain embodiments of the compositions as otherwise described herein, M1 is a mixture of Pt and La, present in a weight ratio of Pt to La within the range of 5:1 to 1 :5, or 4:1 to 1:5, or 3:1 to 1 :5, or 2:1 to 1:5, or 1 :1 to 1:5, or 5:1 to 1:4, or 5:1 to 1:3, or 5:1 to 1:2, or 5:1 to 1:1, or 4:1 to 1:4, or 3:1 to 1:3, or 2:1 to 1:2.

In certain embodiments of the compositions as otherwise described herein, M1 is a mixture of Ir and La, present in a weight ratio of Ir to La within the range of 5:1 to 1:5, or 4:1 to 1:5, or 3:1 to 1:5, or 2:1 to 1:5, or 1:1 to 1:5, or 5:1 to 1:4, or 5:1 to 1:3, or 5:1 to 1:2, or 5:1 to 1:1,or 4:1 to 1:4, or 3:1 to 1:3, or 2:1 to 1:2.

In certain embodiments of the compositions as otherwise described herein, M1 is present in the composition in an amount within the range of 0.005 wt.% to 3.75 wt.%, or 0.005 wt.% to 3.5 wt.%, or 0.005 wt.% to 3.25 wt.%, or 0.005 wt.% to 3 wt.%, or 0.005 wt.% to 2.75 wt.%, or 0.005 wt.% to 2.5 wt.%, or 0.005 wt.% to 2.25 wt.%, or 0.005 wt.% to 2 wt.%, or 0.005 wt.% to 1.75 wt.%, or 0.005 wt.% to 1.5 wt.%, or 0.005 wt.% to 1.25 wt.%, or 0.005 wt.% to 1 wt.%, or 0.005 wt.% to 0.9 wt.%, or 0.005 wt.% to 0.8 wt.%, or 0.005 wt.% to 0.7 wt.%, or 0.005 wt.% to 0.6 wt.%, or 0.005 wt.% to 0.5 wt.%, or 0.01 wt.% to 4 wt.%, or 0.05 wt.% to 4 wt. %, or 0.1 wt.% to 4 wt.%, or 0.2 wt.% to 4 wt.%, or 0.3 wt.% to 4 wt.%, or 0.4 wt.% to 4 wt. %, or 0.5 wt.% to 4 wt.%, or 0.6 wt.% to 4 wt.%, or 0.7 wt.% to 4 wt.%, or 0.8 wt.% to 4 wt. %, or 0.9 wt.% to 4 wt.%, or 1 wt.% to 4 wt.%, or 0.0075 to 3.5 wt.%, or 0.0075 to 3 wt. %, or 0.01 wt.% to 2.5 wt.%, or 0.01 wt.% to 2 wt.%, or 0.01 wt.% to 1.9 wt.%, or 0.01 wt. % to 1.8 wt.%, or 0.01 wt.% to 1.7 wt.%, or 0.01 wt.% to 1.6 wt.%, or 0.01 wt.% to 1 .5 wt. %, or 0.01 wt.% to 1.4 wt.%, or 0.01 wt.% to 1.3 wt.%, or 0.01 wt.% to 1.2 wt.%, or 0.015 wt.% to 1.1 wt.%, or 0.02 wt.% to 1 wt.%, or 0.02 wt.% to 0.9 wt.%, or 0.02 wt.% to 0.8 wt. %, or 0.02 wt.% to 0.7 wt.%, or 0.02 wt.% to 0.6 wt.%, or 0.02 wt.% to 0.5 wt.%, calculated as oxide on a calcined basis.

As described above, in one aspect of the compositions of the disclosure, M2, selected from group 1 of the periodic table of the elements, is present in an amount within the range of 0.05 wt% to 3 wt.%, calculated as oxide on a calcined basis. In certain embodiments of the compositions as otherwise described herein, M2 is selected from lithium, sodium, potassium, and cesium. For example, in certain embodiments of the compositions as otherwise described herein, M2 is potassium. In certain embodiments of the compositions as otherwise described herein, M2 is present in the composition in an amount within the range of 0.05 wt.% to 2.75 wt.%, or 0.05 wt.% to 2.5 wt.%, or 0.05 wt. % to 2.25 wt.%, or 0.05 wt.% to 2 wt.%, or 0.05 wt.% to 1.75 wt.%, or 0.05 wt.% to 1.5 wt.%, or 0.05 wt.% to 1.25 wt.%, or 0.05 wt.% to 1 wt.%, or 0.1 wt.% to 3 wt.%, or 0.25 wt.% to 3 wt.%, or 0.5 wt.% to 3 wt.%, or 0.75 wt.% to 3 wt.%, or 1 wt.% to 3 wt.%, or 1.25 wt.% to 3 wt.%, or 1.5 wt.% to 3 wt.%, or 1.75 wt.% to 3 wt.%, or 2 wt.% to 3 wt.%, or 0.1 wt.% to 2.5 wt.%, or 0.1 wt.% to 2 wt.%, or 0.1 wt.% to 1.75 wt.%, or 0.1 wt.% to 1.5 wt.%, or 0.1 wt.% to 1.25 wt.%, or 0.1 wt.% to 1 wt.%, calculated as oxide on a calcined basis.

In certain embodiments of the compositions as otherwise described herein, the composition further comprises a promoter, M1-B, selected from tin and palladium, present in an amount up to about 0.5 wt.%, e.g., within the range of 0.005 wt% to 0.5 wt.%, calculated as oxide on a calcined basis. For example, in certain embodiments of the compositions as otherwise described herein, M1-B is tin. In certain embodiments of the compositions as otherwise described herein, M1-B is present in the composition in an amount within the range of 0.005 wt.% to 0.4 wt.%, or 0.005 wt.% to 0.3 wt.%, or 0.005 wt.% to 0.2 wt.%, or 0.005 wt.% to 0.1wt.%, or 0.005 wt.% to 0.05 wt.%, or 0.01 wt.% to 0.5 wt.%, or 0.025 wt.% to 0.5 wt.%, or 0.05 wt.% to 0.5 wt.% or 0.075wt. % to 0.5 wt.%, or 0.1 wt.% to 0.5 w.t% or 0.2 wt.% to 0.5wt.%, or 0.3 wt.% to 0.5 wt.%, or 0.0075 wt.% to 0.4 wt.%, or 0.01 wt.% to 0.3 wt.%, or 0.01 wt.% to 0.2 wt.%, calculated as oxide on a calcined basis.

In certain embodiments of the compositions as otherwise described herein, the composition further comprises a promoter, M3, selected from group 2 and group 7-10 of the periodic table of the elements, present in an amount up to about 10 wt.%, e.g., within the range of 0.05 wt% to 10 wt.%, calculated as oxide on a calcined basis. For example, in certain embodiments of the compositions as otherwise described herein, M3 is selected from calcium, strontium, barium, and iron. In certain embodiments of the compositions as otherwise described herein, M3 is present in the composition in an amount within the range of 0.05 wt.% to 9 wt.%, or 0.05 wt.% to 8 wt.%, or 0.05 wt.% to 7 wt.%, or 0.05 wt.% to 6 wt.%, or 0.05 wt.% to 5 wt.%, or 0.1 wt.% to 10 wt.%, or 0.25 wt.% to 10 wt.%, or 0.5 wt.% to 10 wt.%, or 1 wt.% to 10 wt.%, or 2 wt. % to 10 wt.%, or 3 wt.% to 10 wt.%, or 4 wt.% to 10 wt.%, or 5 wt.% to 10 wt.%, or 0.075 wt.% to 9 wt.%, or 0.1 wt.% to 8 wt.%, or 0.1 wt.% to 7 wt.%, or 0.1 wt.% to 6 wt.%, or 0.1 wt.% to 5 wt.%, calculated as oxide on a calcined basis.

For example, in certain embodiments of the compositions as otherwise described herein, gallium oxide is present in the composition in an amount within the range of 1 wt. % to 20 wt.%, 1 wt.% to 17.5 wt.%, or 2 wt.% to 15 wt.%, calculated as Ga₂O₃ on a calcined basis. In certain such embodiments, cerium oxide is present in the composition in an amount within the range of 0.1 wt.% to 10 wt.%, 0.1 wt. % to 5 wt.%, or 0.1 wt.% to 3 wt.%, calculated as CeO₂ on a calcined basis. In certain such embodiments, M1 is platinum, iridium, lanthanum, or a mixture thereof, present in the composition in an amount within the range of 0.01 wt.% to 2 wt.%, 0.015 wt.% to 1.5 wt.%, or 0.02 wt.% to 1 wt.%, calculated as oxide on a calcined basis. In certain such embodiments, M2 is potassium, present in the composition in an amount within the range of 0.05 wt.% to 2 wt.%, 0.075 wt.% to 1.5 wt.%, or 0.1 wt.% to 1 wt.%, calculated as K₂O on a calcined basis. In certain such embodiments, M1-B is tin or palladium, present in an amount within the range of 0.005 wt.% to 0.5wt.%, 0.0075 wt.% to 0.3 wt.%, or 0.01 wt.% to 0.2 wt.%, calculated as SnO₂ or PdO, respectively, on a calcined basis. In certain such embodiments, M3 is selected from Ca, Sr, Ba, and Fe, present in an amount within the range of 0.05 wt.% to 7 wt.%, 0.075 wt.% to 6 wt.%, or 0.1 wt.% to 5 wt.%, calculated as oxide on a calcined basis

As described above, in one aspect of the compositions of the disclosure, S1, selected from alumina, silica, zirconia, titania, or a mixture thereof, is present in an amount within the range of 60 wt% to 99 wt.%, calculated as oxide on a calcined basis. For example, in certain embodiments of the compositions as otherwise described herein, S1 is alumina.

In certain embodiments of the compositions as otherwise described herein, S1 is a mixture of alumina and silica, present in a weight ratio within the range of 0.5:1 to 25:1. The person of ordinary skill in the art will appreciate that, as used herein, a "mixture" of alumina and silica includes homogeneous and heterogeneous mixtures. For example, the mixture of alumina and silica may comprise a covalently bound network including both silicon and aluminum atoms (e.g., -Si-O-Al-), or discrete domains of both silica and alumina. In certain embodiments of the compositions as otherwise described herein, S1 is a mixture of alumina and silica, present in a weight ratio within the range of 0.5:1 to 1 :1, or 1 :1 to 2:1, or 2:1 to 5:1, or 5:1 to 10:1, or 10:1 to 15:1, or 15:1 to 20:1, or 20:1 to 25:1

In certain embodiments of the compositions as otherwise described herein, S1 is a mixture of alumina and zirconia, present in a weight ratio within the range of 1:1 to about 25:1. For example, in certain embodiments of the compositions as otherwise described herein, S1 is a mixture of alumina and zirconia, present in a weight ratio within the range of 1 :1 to 2:1, or 2:1 to 5:1, or 5:1 to 10:1, or 10:1 to 15:1, or 15:1 to 20:1, or 20:1 to 25:1.

In certain embodiments of the compositions as otherwise described herein, S1 is a mixture of alumina and titania, present in a weight ratio within the range of 1.5:1 to 25:1. For example, in certain embodiments of the compositions as otherwise described herein, S1 is a mixture of alumina and titania, present in a weight ratio within the range of 1:5 to 2:1, or 2:1 to 5:1, or 5:1 to 10:1, or 10:1 to 15:1, or 15:1 to 20:1, or 20:1 to 25:1.

In certain embodiments of the compositions as otherwise described herein, S1 is a mixture of alumina, silica, and zirconia, alumina and silica present in a weight ratio within the range of 0.1:1 to 25:1, and alumina and zirconia present in a weight ratio within the range of 90:1 to 40:1. For example, in certain embodiments of the compositions as otherwise described herein, S1 is a mixture of alumina, silica, and zirconia, alumina and silica present in a weight ratio within the range of 0.1 :1 to 0.5:1 , or 0.5:1 to 1 :1, or 1 :1 to 2:1, or 2:1 to 5:1, or 5:1 to 10:1, or 10:1 to 15:1, or 15:1 to 20:1, or 20:1 to 25:1, and alumina and zirconia present in a weight ratio within the range of 90:1 to 80:1, or 80:1 to 70:1, or 70:1 to 60:1, or 60:1 to 50:1, or 50:1 to 40:1.

The person of ordinary skill in the art will appreciate that the catalyst composition may, in some embodiments as otherwise described herein, be substantially free of chromium. For example, in certain embodiments of the compositions as otherwise described herein, the catalyst composition includes less than 1 wt.%, or less than 0.9 wt. %, or less than 0.8 wt.%, or less than 0.7 wt.%, or less than 0.6 wt.%, or less than 0.5 wt.%, or less than 0.4 wt.%, or less than 0.3 wt.%, or less than 0.2 wt.%, or less than 0.1 wt.%, or less than 0.05 wt.%, or less than 0.01 wt.% of chromium, calculated as Cr₂O₃.

The person of ordinary skill in the art will appreciate that the catalyst composition may, in some embodiments as otherwise described herein, be substantially free of each of the lanthanides other than lanthanum and cerium. For example, in certain embodiments of the compositions as otherwise described herein, the catalyst composition includes less than about 2 wt.%, or less than 1.5 wt.%, or less than 1 wt.%, or less than 0.9 wt.%, or less than 0.8 wt.%, or less than 0.7 wt.%, or less than 0.6 wt. %,or less than 0.5 wt.%, or less than 0.4 wt.%, or less than 0.3 wt.%, or less than 0.2 wt.%, or less than 0.1 wt.%, or less than 0.05 wt.%, or less than 0.01 wt.% of the lanthanides other than lanthanum and cerium, calculated as La₂O₃ and CeO₂, respectively.

In certain desirable embodiments of the compositions as otherwise described herein, the total amount of the gallium oxide, cerium oxide, promoters (e.g., M1, M1-B, M2, and M3 ), and support (e.g., S1) is at least 80 wt.%, or at least 85 wt.%, or at least 90 wt.%, or at least 95 wt.%, or at least 97.5 wt.%, or at least 99 wt.% of the composition (i.e., calculated on an as-calcined oxide basis).

In certain desirable embodiments of the compositions as otherwise described herein, S1 comprises a covalent network structure, throughout which structure one or more of the gallium oxide, cerium oxide, and promoters (e.g., M1, M1-B, M2, and M3) are dispersed. For example, in certain embodiments of the compositions as otherwise described herein, S1 comprises the product of a hydrolysis-polycondensation reaction of one or more metal oxy compounds, e.g., performed in the presence of a gallium source, a cerium source, and one or more promoter sources. In certain such embodiments, the metal oxy compounds include metal alkoxides (e.g., aluminum isopropoxide, tetraethyl orthosilicate, titanium n-butoxide, and zirconium n-propoxide), metal oxynitrates (e.g., zirconyl nitrate), or metal hydroxides (e.g., aluminum hydroxide).

Another aspect of the disclosure is a method for preparing a dehydrogenation catalyst composition as described herein. The method includes providing a gallium source, a cerium source, an M1 source, an M2 source, an M3 source, and an S1 source, forming a solid from the solution (e.g., by a hydrolysis-polycondensation reaction), and calcining the solid so formed. The amounts and identities of the various components (e.g., promoters M1, M1-B, M2, M3 and support S1) can be as otherwise described above with respect to the catalyst compositions of the disclosure.

In certain embodiments of the methods as otherwise described herein, the gallium source is a gallium salt. For example, in certain embodiments of the methods as otherwise described herein, the gallium source is gallium nitrate or gallium acetylacetonate.

In certain embodiments of the methods as otherwise described herein, the cerium source is a cerium salt. For example, in certain embodiments of the methods as otherwise described herein, the cerium source is cerium nitrate.

In certain embodiments of the methods as otherwise described herein, the M1 source is a salt. For example, in certain embodiments of the methods as otherwise described herein, the M1 source is tetraamineplatinum(ll) nitrate, hexachloroplatinate, iridium(IV) chloride, or lanthanum(III) nitrate.

In certain embodiments of the methods as otherwise described herein, the M2 source is a salt. For example, in certain embodiments of the methods as otherwise described herein, the M2 source is potassium nitrate.

In certain embodiments of the methods as otherwise described herein, the aqueous solution further comprises an M1-B source. In certain such embodiments, the M1-B source is a salt. For example, in certain embodiments of the methods as otherwise described herein, the M1-B source is tin(IV) chloride or palladium nitrate.

In certain embodiments of the methods as otherwise described herein, the aqueous solution further comprises an M3 source. In certain such embodiments, the M3 source is a salt. For example, in certain embodiments of the methods as otherwise described herein, the M3 source is calcium nitrate, strontium nitrate, barium nitrate, or iron(III) nitrate.

As described above, in one aspect of the methods of the disclosure, the method includes providing an aqueous solution comprising an S1 source. In certain embodiments of the methods as otherwise described herein, the S1 source includes one or more metal compounds selected from metal oxides and metal salts. For example, in certain embodiments of the methods as otherwise described herein, the S1 source includes one or more metal oxides selected from silica, alumina, or lanthania. In another example, in certain embodiments of the methods as otherwise described herein, the S1 source includes one or more metal salts selected from zirconium carbonate, aluminum nitrate, sodium silicate, or lanthanum nitrate.

In certain embodiments of the methods as otherwise described herein, the S1 source includes one or more metal oxy compounds selected from metal alkoxides, metal hydroxides, and metal oxynitrates. For example, in certain embodiments of the methods as otherwise described herein, the S1 source includes one or more of an aluminum alkoxide (e.g., aluminum isopropoxide), a silicon alkoxide (e.g., tetraethyl orthosilicate), a titanium alkoxide (e.g., titanium n-butoxide), and a zirconium alkoxide (e.g., zirconium n-propoxide). In another example, in certain embodiments of the methods as otherwise described herein, the S1 source includes an aluminum hydroxide (e.g., bayerite or boehmite). In another example, in certain embodiments of the methods as otherwise described herein, the S1 source includes a zirconium oxynitrate.

As described above, in one aspect of the methods of the disclosure, the method includes forming a solid from the solution, for example, by a hydrolysis-polycondensation reaction. This can be performed, for example, using conventional processes, including sol-gel processes familiar to the person of ordinary skill in the art. The solid can be, for example, in the form of a monolithic solvent, or in the form of particles, (e.g., as a slurry). In certain embodiments of the methods as otherwise described herein, forming the solid comprises performing a hydrolysis-polycondensation on at least a portion of the S1 source to provide a covalent network structure. For example, in certain embodiments of the methods as otherwise described herein, forming the solid comprises performing a hydrolysis-polycondensation on at least a portion of a metal oxy compound of the S1 source, e.g., aluminum alkoxide, silicon alkoxide, titanium alkoxide, zirconium alkoxide, aluminum hydroxide, zirconium hydroxide, zirconyl nitrate, etc. In certain embodiments of the methods as otherwise described herein, forming the solid is acid-catalyzed. In certain embodiments of the methods as otherwise described herein, forming the solid provides a covalent network structure, throughout which structure one or more of the gallium source, cerium source, M1 source, M1-B source, M2 source, and M3 source are dispersed.

In certain embodiments of the methods as otherwise described herein, the method includes heating the aqueous solution. For example, in certain embodiments of the methods as otherwise described herein, the aqueous solution is heated to a temperature within the range of about 60 °C to about 100 °C, or about 70 °C to about 95 °C, or about 80 °C to about 90 °C to form the solid.

In certain embodiments of the methods as otherwise described herein, the method includes aging the aqueous solution after acid is added. For example, in certain embodiments of the methods as otherwise described herein, the aqueous solution is aged to a time of period within the range of about 1 hour to about 3 hours, or about 1.5 hours to about 5 hours, or about 2 hours to about 7 hours to form the gel/slurry.

As described above, the method includes calcining the solid (e.g., after removing the solvent from the solid, for example, by filtration and drying). As the person of ordinary skill in the art will appreciate, further condensation reaction can occur during the calcining. In certain embodiments of the methods as otherwise described herein, the solid is calcined at a temperature within the range of about 300 °C to about 900 °C. For example, in certain embodiments, the solid is calcined at a temperature within the range of about 350 °C to about 900 °C, or about 400 °C to about 900 °C, or about 450 °C to about 900 °C, or about 500 °C to about 900 °C, or about 550 °C to about 900 °C, or about 300 °C to about 850 °C, or about 300 °C to about 800 °C, or about 300 °C to about 750 °C, or about 300 °C to about 700 °C, or about 300 °C to about 650 °C, or about 350 °C to about 850 °C, or about 400 °C to about 800 °C, or about 450 °C to about 750 °C.

In certain embodiments of the methods as otherwise described herein, the solid is calcined for a period of time within the range of about 5 min. to about 12 hr. For example, in certain embodiments of the methods as otherwise described herein, the solid is calcined for a period of time within the range of about 10 min. to about 12 hr., or about 15 min. to about 12 hr., or about 20 min. to about 12 hr., or about 30 min. to about 12 hr., or about 45 min. to about 12 hr., or about 1 hr. to about 12 hr., or about 1.5 hr. to about 12 hr., or about 2 hr. to about 12 hr., or about 5 min. to about 11 hr., or about 5 min. to about 10 hr., or about 5 min. to about 9 hr., or about 5 min. to about 8hr., or about 5 min. to about 7.5 hr., or about 5 min. to about 7 hr., or about 5 min. to about 6.5 hr., or about 5 min. to about 6 hr., or about 5 min. to about 5.5 hr., or about 5 min. to about 5 hr., or about 30 min. to about 11 hr., or about 1 hr. to about 10 hr., or about 1.5 hr. to about 9 hr., or about 2 hr. to about 8 hr.

In certain embodiments of the methods as otherwise described herein, the solid is dried before calcination. In certain embodiments of the methods as otherwise described herein, the solid is dried at a temperature within the range of about 80 °C to about 240 °C. For example, in certain embodiments of the methods as otherwise described herein, the solid is dried at a temperature within the range of about 80 °C to about 220 °C, or about 80 °C to about 200 °C, or about 80 °C to about 180 °C, or about 100 °C to about 240 °C, or about 120 °C to about 240 °C, or about 140 °C to about 240 °C, or about 100 °C to about 220 °C, or about 120 °C to about 200 °C, or about 140 °C to about 180 °C.

In certain embodiments of the methods as otherwise described herein, the solid is dried for a period of time within the range of about 4 hr. to about 36 hr. For example, in certain embodiments of the methods as otherwise described herein, the solid is dried for a period of time within the range of about 4 hr. to about 30 hr., or about 4 hr. to about 24 hr., or about 4 hr. to about 22 hr., or about 4 hr. to about 20 hr., or about 6 hr. to about 36 hr., or about 8 hr. to about 36 hr., or about 10 hr. to about 36 hr., or about 12 hr. to about 36 hr., or about 6 hr. to about 30 hr., or about 8 hr. to about 24 hr., or about 10 hr. to about 22 hr., or about 12 hr. to about 20 hr.

Another aspect of the disclosure is a catalyst composition prepared by a method as described herein.

Advantageously, the present inventors have determined that use of catalyst compositions described herein can catalyze a hydrocarbon dehydrogenation reaction at an efficiency comparable to or better than conventional, commercially available catalyst materials.

The compositions described herein are especially useful in hydrocarbon dehydrogenation reactions. Accordingly, another aspect of the disclosure is a method for dehydrogenating alkanes that includes contacting a hydrocarbon feed with a catalyst composition as described herein under conditions sufficient to cause hydrocarbon dehydrogenation.

In some embodiments of the dehydrogenation methods as otherwise described herein, the hydrocarbon feed comprises one or more C₃-C₅ alkanes. For example, in certain embodiments of the dehydrogenation methods as otherwise described herein, the hydrocarbon feed comprises propane.

The contacting of the feed with the catalyst compositions described herein can be conducted in a variety of ways familiar to the person of ordinary skill in the art. Conventional equipment and processes can be used in conjunction with the catalyst compositions of the disclosure to provide beneficial performance. Thus, the catalyst may be contained in one bed within a reactor vessel or divided up among a plurality of beds within a reactor. The reaction system may contain one or more reaction vessels in series. The feed to the reaction zone can flow vertically upwards, or downwards through the catalyst bed in a typical plug flow reactor, or horizontally across the catalyst bed in a radial flow type reactor.

The contacting of the feed with the catalyst composition can be performed using conventional methods. For example, the feed may be introduced into the reaction zone containing the catalyst composition at a constant rate, or alternatively, at a variable rate.

In certain embodiments of the dehydrogenation methods as otherwise described herein, the feed is contacted with the provided catalyst composition at a liquid hourly space velocity (LHSV) within the range of about 0.5 h⁻¹ to about 4 h⁻¹. For example, in certain embodiments of the dehydrogenation methods as otherwise described herein, the feed is contacted with the provided catalyst composition at a liquid hourly space velocity of about 0.75 h⁻¹ to about 4 h⁻¹, or about 1 h⁻¹ to about 4 h⁻¹, or about 1.25 h⁻¹ to about 4 h⁻¹, or about 1.5 h⁻¹ to about 4 h⁻¹, or about 0.5 h⁻¹ to about 3.75 h⁻¹, or about 0.5 h⁻¹ to about 3.5 h⁻¹, or about 0.5 h⁻¹ to about 3.25 h⁻¹, or about 0.5 h⁻¹ to about 3 h⁻¹, or about 0.5 h⁻¹ to about 2.75 h⁻¹, or about 0.5 h⁻¹ to about 2.5 h⁻¹, or about 0.75 h⁻¹ to about 3.5 h⁻¹, or about 1 h⁻¹ to about 3 h⁻¹, or about 1.25 h⁻¹ to about 2.75 h⁻¹, or about 1.5 h⁻¹ to about 2.5 h⁻¹.

In certain embodiments of the dehydrogenation methods as otherwise described herein, the method is carried out at a temperature within the range of about 400 °C to about 750 °C. For example, in certain embodiments of the dehydrogenation methods as otherwise described herein, the method is carried out at a temperature within the range of about 400 °C to about 700 °C, or about 400 °C to about 650 °C, or about 400 °C to about 600 °C, or about 400 °C to about 550 °C, or about 450 °C to about 750 °C, or about 500 °C to about 750 °C, or about 550 °C to about 750 °C, or about 600 °C to about 750 °C, or about 450 °C to about 700 °C, or about 500 °C to about 650 °C.

In certain embodiments of the dehydrogenation methods as otherwise described herein, the method is carried out at a pressure within the range of about 0.1 bar to about 1 bar. For example, in certain embodiments of the dehydrogenation methods as otherwise described herein, the methods is carried out at a pressure within the range of about 0.1 bar to about 0.9 bar, or about 0.1 bar to about 0.8 bar, or about 0.1 bar to about 0.7 bar, or about 0.1 bar to about 0.6 bar, or about 0.1 bar to about 0.5 bar, or about 0.2 bar to about 1 bar, or about 0.3 bar to about 1 bar, or about 0.4 bar to about 1 bar, or about 0.5 bar to about 1 bar, or about 0.2 bar to about 0.9 bar, or about 0.3 bar to about 0.8 bar, or about 0.4 bar to about 0.7 bar.

### EXAMPLES

The Examples that follow are illustrative of specific embodiments of the invention, and various uses thereof. They are set forth for explanatory purposes only, and are not to be taken as limiting the invention.

### Example 1. Catalyst Preparation

Catalyst **A1** was prepared via sol-gel synthesis: 1.35 g Ga(NO₃)₃, 0.082 g KNO₃, 0.38 g Ce(NO₃)₃·6H₂O, and 0.044 g La(NO3)₃·6H2O were mixed together with 424.1 g DI water in a container at room temperature, then heated to 90 °C for 15 min. to reach a milk-like colloidal solution, to which 48.2 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A2** was prepared via sol-gel synthesis: 1.32 g Ga(NO₃)₃, 0.09 g KNO₃, 0.38 g Ce(NO₃)₃·6H₂O, and 0.02 g Pt(NH₃)₄(NO3)₂ were mixed together with 424.3 g DI water in a container at room temperature, then heated to 90 °C for 15 min. to reach a milk-like colloidal solution, to which 48.1 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A3** was prepared via sol-gel synthesis: 5.4 g Ga(NO₃)₃, 0.32 g KNO₃, 1.53 g Ce(NO₃)₃·6H₂O, and 0.084 g Pt(NH₃)₄(NO3)₂ were mixed together with 1696 g DI water in a closed container at room temperature, then heated to 90 °C for 15 min. to reach a milk-like colloidal solution, to which 192 g aluminum isopropoxide was slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A4** was prepared via sol-gel synthesis: 10.74 g Ga(NO₃)₃, 0.32 g KNO₃, 1.56 g Ce(NO₃)₃·6H₂O, 0.083 g Pt(NH₃)₄(NO3)₂, and 0.085 g SnCl₄·4H₂O were mixed together with 1696 g DI water in a container at room temperature, then heated to 90 °C for 15 min. to reach a milk-like colloidal solution, to which 192 g aluminum isopropoxide was slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A5** was prepared via sol-gel synthesis: 5.40 g Ga(NO₃)₃, 0.34 g KNO₃, 1.53 g Ce(NO₃)₃·6H₂O, 0.083 g IrCl₃, and 0.088 g SnCl₄·4H₂O were mixed together with 1696 g DI water in a closed container at room temperature, then heated to 90 °C for 15 min. to reach a homogenous solution, to which 192 g aluminum isopropoxide was slowly added. The mixture was stirred at 90 °C for at least 30 min., then an aqueous solution containing 18.0 g HNO₃ and 5.5 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A6** was prepared via sol-gel synthesis: 10.73 g Ga(NO₃)₃, 0.32 g KNO₃, 1.50 g Ce(NO₃)₃·6H₂O, 0.086 g IrCl₃, and 0.086 g SnCl₄·4H₂O were mixed together with 1696 g DI water in a container at room temperature, then heated to ~90 °C for 15 min. to reach a homogenous solution, to which 192 g aluminum isopropoxide was slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 5 hours to provide a concentrated slurry/gel. The composition was dried in air at 120 °C for 16 hours and calcined at 600 °C in air for 4 hours.

Catalyst **A7** was prepared via sol-gel synthesis: 10.76 g Ga(NO₃)₃, 0.32 g KNO₃, 1.52 g Ce(NO₃)₃·6H₂O, 0.081 g IrCl₃, and 0.087 g SnCl₄·H₂O were mixed together with 1696 g DI water in a closed container at room temperature, then heated to ~90 °C for 15 min. to reach a homogenous solution, to which 144.4 g aluminum isopropoxide and 48.0 g tetraethylorthosilicate were slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A8** was prepared via sol-gel synthesis: 21.5 g Ga(NO₃)₃, 0.33 g KNO₃, 1.53 g Ce(NO₃)₃·6H₂O, 0.081 g IrCl₃, and 0.088 g SnCl₄·H₂O were mixed together with 1696 g DI water in a container at room temperature, then heated to ~90 °C for 15 min. to reach a homogenous solution, to which 192 g aluminum isopropoxide was slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 7 hours to provide a concentrated slurry/gel. The composition was dried in air at 120 °C for 16 hours and calcined at 600 °C in air for 4 hours.

Catalyst **A9** was prepared via sol-gel synthesis: 2.1 g Ga(NO₃)₃, 0.083 g KNO₃, 0.40 g Ce(NO₃)₃·6H₂O, 0.046 g IrCl₃, and 1.49 g Mg(NO₃)₂·6H₂O were mixed together with 424 g DI water in a container at room temperature, then heated to ~90 °C for 15 min. to reach a homogenous solution, to which 48 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A10** was prepared via sol-gel synthesis: 2.7 g Ga(NO₃)₃, 0.12 g KNO₃, 0.38 g Ce(NO₃)₃·6H₂O, 0.028 g IrCl₃, and 0.029 g SnCl₄·4H₂O were mixed together with 424 g DI water in a container at room temperature, then heated to ~90 °C for 15 min. to reach a homogenous solution, to which 48 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 2 hours.

Catalyst **A11** was prepared via sol-gel synthesis: 10.73 g Ga(NO₃)₃, 0.32 g KNO₃, 1.52 g Ce(NO₃)₃·6H₂O, 0.082 g IrCl₃, 6.02 g Mg(NO₃)₂·6H₂O, and 0.081 g SnCl₄·4H₂O were mixed together with 1696.1 g DI water in a container at room temperature, then heated to ~90 °C for 15 min. to reach a homogenous solution, to which 192.3 g aluminum isopropoxide was slowly added. The mixture was stirred at 90 °C for at least 30 min., then an aqueous solution containing 17.9 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 2 hours.

Catalyst **A12** was prepared via sol-gel synthesis: 8.28 g Ga(NO₃)₃, 0.33 g KNO₃, 1.56 g Ce(NO₃)₃·6H₂O, 0.155 g IrCl₃, and 6.05 g Mg(NO₃)₂·6H₂O were mixed together with 1696.0 g DI water in a container at room temperature, then heated to ~90 °C for 15 min. to reach a homogenous solution, to which 192.1 g aluminum isopropoxide was slowly added. The mixture was stirred at 90 °C for at least 30 min., then an aqueous solution containing 18.0 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 2 hours.

Catalyst **A13** was prepared via sol-gel synthesis: 2.1 g Ga(NO₃)₃, 0.08 g KNO₃, 0.38 g Ce(NO₃)₃·6H₂O, 0.039 g IrCl₃, and 2.24 g Mg(NO₃)₂·6H₂O were mixed together with 424.1 g DI water in a container at room temperature, then heated to ~90 °C for 15 min. to reach a homogenous solution, to which 48.0 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 2 hours.

Catalyst **A14** was prepared via sol-gel synthesis: 2.1 g Ga(NO₃)₃, 0.083 g KNO₃, 0.39 g Ce(NO₃)₃·6H₂O, 0.039 g IrCl₃, and 1.5 g Ca(NO₃)₂ were mixed together with 424.1 g DI water in a container at room temperature, then heated to ~90 °C for 15 min. to reach a homogenous solution, to which 48.2 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 2 hours.

Catalyst **A15** was prepared via sol-gel synthesis: 8.3 g Ga(NO₃)₃, 0.33 g KNO₃, 1.55 g Ce(NO₃)₃·6H₂O, 0.082 g Pt(NH₃)₄(NO3)₂, and 1.55 g Ca(NO₃)₂ were mixed together with 1696.1 g DI water in a container at room temperature, then heated to ~90 °C for 15 min. to reach a milk-like solution, to which 192.2 g aluminum isopropoxide was slowly added. The mixture was stirred at 90 °C for at least 30 min., then an aqueous solution containing 17.9 g HNO₃ and 5.5 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A16** was prepared via sol-gel synthesis: 8.3 g Ga(NO₃)₃, 0.33 g KNO₃, 1.55 g Ce(NO₃)₃·6H₂O, 0.082 g Pt(NH₃)₄(NO3)₂, and 1.83 g Mg(NO₃)₂·6H₂O were mixed together with 1696.0 g DI water in a container at room temperature, then heated to ~90 °C for 15 min. to reach a milk-like colloidal solution, to which 144.6 g aluminum isopropoxide and 48.7 g tetraethyl orthosilicate were slowly added. The mixture was stirred at 90 °C for at least 30 min., then an aqueous solution containing 17.9 g HNO₃ and 5.5 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A17** was prepared via sol-gel synthesis: 5.36 g Ga(NO₃)₃, 0.085 g KNO₃, 0.39 g Ce(NO₃)₃·6H₂O, 0.023 g SnCl₄·4H₂O 0.021 g Pt(NH₃)₄(NO3)₂, and 1.53 g Mg(NO₃)₂·6H₂O were mixed together with 423.9 g DI water in a container at room temperature, then heated to ~90 °C for 15 min. to reach a milk-like colloidal solution, to which 35.9 g aluminum isopropoxide and 12.0 g zirconyl nitrate were slowly added. The mixture was stirred at 90 °C for at least 30 min., then an aqueous solution containing 4.5 g HNO₃ and 1.5 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A18** was prepared via sol-gel synthesis: 8.5 g Ga(NO₃)₃, 0.52 g KNO₃, 1.72 g Ce(NO₃)₃·6H₂O, 1.82 g Pt(NH₃)₄(NO3)₂, and 1.89 g Mg(NO₃)₂·6H₂O were mixed together with 1696 g DI water in a container at room temperature, then heated to ~90 °C for 15 min. to reach a milk-like colloidal solution, to which 144.5 g aluminum isopropoxide and 48.7 g tetraethyl orthosilicate were slowly added. The mixture was stirred at 90 °C for at least 30 min., then an aqueous solution containing 17.9 g HNO₃ and 5.5 g DI water was added to the mixture. The final mixture was further heated at 86-90 °C with vigorous stirring for ~ 6 hours to provide a concentrated slurry/gel . The composition was dried in air at 120 °C for 16 hours and calcined at 600 °C in air for 4 hours.

Catalyst **A19** was prepared via sol-gel synthesis: 8.4 g Ga(NO₃)₃, 0.50 g KNO₃, 1.59 g Ce(NO₃)₃·6H₂O, 1.83 g Pt(NH₃)₄(NO3)₂, and 0.71 g Mn(NO₃)₂·4H₂O were mixed together with 1696.7 g DI water in a container at room temperature, then heated to ~90 °C for 15 min. to reach a milk-like colloidal solution, to which 144.5 g aluminum isopropoxide and 50.8 g tetraethyl orthosilicate were slowly added. The mixture was stirred at 90 °C for at least 30 min., then an aqueous solution containing 17.98 g HNO₃ and 5.57 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A20** was prepared via sol-gel synthesis: 8.29 g Ga(NO₃)₃, 0.46 g KNO₃, 1.53 g Ce(NO₃)₃·6H₂O, 1.81 g Pt(NH₃)₄(NO3)₂, and 5.98 g Ca(NO₃)₂ were mixed together with 1696.7 g DI water in a container at room temperature, then heated to ~90 °C for 15 min. to reach a milk-like colloidal solution, to which 144.5 g aluminum isopropoxide and 52.8 g tetraethyl orthosilicate were slowly added. The mixture was stirred at 90 °C for at least 30 min., then an aqueous solution containing 17.95 g HNO₃ and 5.42 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A21** was prepared via sol-gel synthesis: 21.76 g Ga(NO₃)₃, 0.45 g KNO₃, 1.52 g Ce(NO₃)₃·6H₂O, 1.82 g IrCl3, 0.1 g SnCl₄·4H₂O, and 1.82 g Mg(NO₃)₂·6H₂O were mixed together with 1696.7 g DI water in a container at room temperature, then heated to ~90 °C for 15 min. to reach a homogenous solution, followed by slow addition of 182.9 g aluminum isopropoxide and 9.62 g tetraethyl orthosilicate. The mixture was stirred at 90 °C for at least 30 min., then an aqueous solution containing 18.0 g HNO₃ and 5.38 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A22** was prepared via sol-gel synthesis: 2.09 g Ga(NO₃)₃, 0.087 g KNO₃, 3.18 g Ce(NO₃)₃·6H₂O, 0.051 g IrCI3, 2.33 g ZrO(NO3), and 1.56 g Mg(NO₃)₂·6H₂O were mixed together with 428.3 g DI water in a container at room temperature, then heated to ~90 °C for 15 min. to reach a homogenous solution, to which 38.8 g aluminum isopropoxide and 9.62 g tetraethyl orthosilicate were slowly added. The mixture was stirred at 90 °C for at least 30 min., then an aqueous solution containing 4.42 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A23** was prepared via sol-gel synthesis: 2.07 g Ga(NO₃)₃, 0.12 g KNO₃, 0.39 g Ce(NO₃)₃·6H₂O, 0.027 g Pt(NH₃)₄(NO3)₂, and 1.51 g Mg(NO₃)₂·6H₂O were mixed together with 424.4 g DI water in a container at room temperature, then heated to ~90 °C for 15 min. to reach a milk-like colloidal solution, to which 48.1 g aluminum isopropoxide and 2.60 g ZrO(NO3)2 were slowly added. The mixture was stirred at 90 °C for at least 30 min., then an aqueous solution containing 4.58 g HNO₃ and 1.4 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A24** was prepared via sol-gel synthesis: 8.34 g Ga(NO₃)₃, 0.33 g KNO₃, 1.57 g Ce(NO₃)₃·6H₂O, 0.082 g Pt(NH₃)₄(NO3)₂, and 3.64 g Mg(NO₃)₂·6H₂O were mixed together with 1696.1 g DI water in a container at room temperature, then heated to ~90 °C for 15 min. to reach a milk-like colloidal solution, to which 144.5 g aluminum isopropoxide and 48.65 g tetraethyl orthosilicate were slowly added. The mixture was stirred at 90 °C for at least 30 min., then an aqueous solution containing 17.96 g HNO₃ and 5.50 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A25** was prepared via sol-gel synthesis: 2.07 g Ga(NO₃)₃, 0.12 g KNO₃, 0.39 g Ce(NO₃)₃·6H₂O, 0.024 g Pt(NH₃)₄(NO3)₂, and 1.50 g Mg(NO₃)₂·6H₂O were mixed together with 424.1 g DI water in a container at room temperature, then heated to ~90 °C for 15 min. to reach a milk-like colloidal solution, to which 48.0 g aluminum isopropoxide and 5.8 g titanium n-butoxide were slowly added. The mixture was stirred at 90 °C for at least 30 min., then an aqueous solution containing 4.64 g HNO₃ and 1.5 0 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A26** was prepared via sol-gel synthesis: 4.14 g Ga(NO₃)₃, 0.12 g KNO₃, 0.48 g Ce(NO₃)₃·6H₂O, 0.023 g Pt(NH₃)₄(NO3)₂, and 1.50 g Mg(NO₃)₂·6H₂O were mixed together with 424.1 g DI water in a container at room temperature, then heated to ~90 °C for 15 min. to reach a mil-like colloidal solution, to which 48.0 g aluminum isopropoxide and 2.6 g zirconyl nitrate were slowly added. The mixture was stirred at 90 °C for at least 30 min., then an aqueous solution containing 4.6 g HNO₃ and 1.5 g DI water was added to the mixture. The final mixture was further heated at 86-90 °C with vigorous stirring for ~ 3 hours to provide a concentrated slurry/gel. The composition was dried in air at 120 °C for 16 hours and calcined at 600 °C in air for 4 hours.

Catalyst **A27** was prepared via sol-gel synthesis: 8.25 g Ga(NO₃)₃, 0.47 g KNO₃, 1.58 g Ce(NO₃)₃·6H₂O, 0.081 g Pt(NH₃)₄(NO3)₂, and 3.95 g Ca(NO₃)₂ were mixed together with 1697 g DI water in a container at room temperature, then heated to ~90 °C for 15 min. to reach a milk-like colloidal solution, to which 144.6 g aluminum isopropoxide and 53.6 g tetraethyl orthosilicate were slowly added. The mixture was stirred at 90 °C for at least 30 min., then an aqueous solution containing 17.97 g HNO₃ and 5.43 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A28** was prepared via sol-gel synthesis: 8.28 g Ga(NO₃)₃, 0.33 g KNO₃, 1.54 g Ce(NO₃)₃·6H₂O, 0.083 g Pt(NH₃)₄(NO3)₂, and 1.84 g Mg(NO₃)₂·6H₂O were mixed together with 1697 g DI water in a container at room temperature, then heated to ~90 °C for 15 min. to reach a milk-like colloidal solution, to which 144.7 g aluminum isopropoxide and 17.94 g tetraethyl orthosilicate were slowly added. The mixture was stirred at 90 °C for at least 30 min., then an aqueous solution containing 17.94 g HNO₃ and 5.44 g DI water was added to the mixture. The final mixture was further heated at 86-90 °C with vigorous stirring for ~ 6 hours to obtain concentrated slurry/gel. The composition was dried in air at 120 °C for 16 hours and calcined at 600 °C in air for 4 hours.

Catalyst **A29** was prepared via sol-gel synthesis: 8.25 g Ga(NO₃)₃, 0.33 g KNO₃, 1.56 g Ce(NO₃)₃·6H₂O, 0.083 g Pt(NH₃)₄(NO3)₂, and 1.83 g Mg(NO₃)₂·6H₂O were mixed together with 1696 g DI water in a container at room temperature, then heated to ~90 °C for 15 min. to reach a milk-like colloidal solution, to which 125.3 g aluminum isopropoxide and 69.4 g tetraethyl orthosilicate were slowly added. The mixture was stirred at 90 °C for at least 30 min., then an aqueous solution containing 17.99 g HNO₃ and 5.47 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A30** was prepared via sol-gel synthesis: 8.26 g Ga(NO₃)₃, 0.33 g KNO₃, 1.55 g Ce(NO₃)₃·6H₂O, 0.082 g Pt(NH₃)₄(NO3)₂, and 1.82 g Mg(NO₃)₂·6H₂O were mixed together with 1697 g DI water in a container at room temperature, then heated to ~90 °C for 15 min. to reach a milk-like colloidal solution, to which 173.0 g aluminum isopropoxide and 9.57 g ZrO(NO3)2 were slowly added. The mixture was stirred at 90 °C for at least 30 min., then an aqueous solution containing 17.97 g HNO₃ and 5.52 g DI water was added to the mixture. The final mixture was further heated at 86-90 °C with vigorous stirring for ~ 6 hours to provide a concentrated slurry/gel . The composition was dried in air at 120 °C for 16 hours and calcined at 600 °C in air for 4 hours.

Catalyst **A31** was prepared via sol-gel synthesis: 8.25 g Ga(NO₃)₃, 0.33 g KNO₃, 1.55 g Ce(NO₃)₃·6H₂O, 0.082 g Pt(NH₃)₄(NO3)₂, and 1.84 g Mg(NO₃)₂·6H₂O were mixed together with 1696 g DI water in a container at room temperature, then heated to ~90 °C for 15 min. to reach a milk-like colloidal solution, to which 106.2 g aluminum isopropoxide and 87.0 g tetraethyl orthosilicate were slowly added. The mixture was stirred at 90 °C for at least 30 min., then an aqueous solution containing 17.95 g HNO₃ and 5.49 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A32** was prepared via sol-gel synthesis: 8.31 g Ga(NO₃)₃, 0.33 g KNO₃, 1.54 g Ce(NO₃)₃·6H₂O, 0.082 g Pt(NH₃)₄(NO3)₂, and 2.01 g Mg(NO₃)₂·6H₂O were mixed together with 1697 g DI water in a container at room temperature, then heated to ~90 °C for 15 min. to reach a milk-like colloidal solution, to which 153.8 g aluminum isopropoxide and 18.71 g ZrO(NO3)2 were slowly added. The mixture was stirred at 90 °C for at least 30 min., then an aqueous solution containing 17.95 g HNO₃ and 5.50 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A33** was prepared via sol-gel synthesis: 8.32 g Ga(NO₃)₃, 0.33 g KNO₃, 1.55 g Ce(NO₃)₃·6H₂O, 0.083 g Pt(NH₃)₄(NO3)₂, and 1.83 g Mg(NO₃)₂·6H₂O were mixed together with 1696 g DI water in a container at room temperature, then heated to ~90 °C for 15 min. to reach a milk-like colloidal solution, to which 134.6 g aluminum isopropoxide and 28.2 g ZrO(NO3)2 were slowly added. The mixture was stirred at 90 °C for at least 30 min., then an aqueous solution containing 17.94 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A34** was prepared via sol-gel synthesis: 8.31 g Ga(NO₃)₃, 0.33 g KNO₃, 1.57 g Ce(NO₃)₃·6H₂O, 0.084 g Pt(NH₃)₄(NO3)₂, and 1.83 g Mg(NO₃)₂·6H₂O were mixed together with 1697 g DI water in a container at room temperature, then heated to ~90 °C for 15 min. to reach a milk-like colloidal solution, to which 172.8 g aluminum isopropoxide and 21.5 g titanium n-butoxide were slowly added. The mixture was stirred at 90 °C for at least 30 min., then an aqueous solution containing 17.92 g HNO₃ and 5.4 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A35** was prepared via sol-gel synthesis: 8.28 g Ga(NO₃)₃, 0.32 g KNO₃, 1.56 g Ce(NO₃)₃·6H₂O, 0.082 g Pt(NH₃)₄(NO3)₂, and 1.83 g Mg(NO₃)₂·6H₂O were mixed together with 1697 g DI water in a container at room temperature, then heated to ~90 °C for 15 min. to reach a milk-like colloidal solution, to which 153.7 g aluminum isopropoxide and 43.3 g titanium n-butoxide were slowly added. The mixture was stirred at 90 °C for at least 30 min., then an aqueous solution containing 17.98 g HNO₃ and 5.5 g DI water was added to the mixture. The final mixture was further heated at 86-90 °C with vigorous stirring for ~ 6 hours to provide a concentrated slurry/gel. The composition was dried in air at 120 °C for 16 hours and calcined at 600 °C in air for 4 hours.

Catalyst **A36** was prepared via sol-gel synthesis: 8.28 g Ga(NO₃)₃, 0.33 g KNO₃, 1.58 g Ce(NO₃)₃·6H₂O, 0.084 g Pt(NH₃)₄(NO3)₂, and 1.83 g Mg(NO₃)₂ were mixed together with 1696 g DI water in a container at room temperature, then heated to ~90 °C for 15 min. to reach a milk-like colloidal solution, to which 144.6 g aluminum isopropoxide and 49.6 g tetraethyl orthosilicate were slowly added. The mixture was stirred at 90 °C for at least 30 min., then an aqueous solution containing 17.98 g HNO₃ and 5.4 g DI water was added to the mixture The final mixture was further heated at 86-90 °C with vigorous stirring for- 6 hours to provide a concentrated slurry/gel. The composition was dried in air at 120 °C for 16 hours and calcined at 600 °C in air for 4 hours.

Catalyst **A37** was prepared via sol-gel synthesis: 8.27 g Ga(NO₃)₃, 0.46 g KNO₃, 1.56 g Ce(NO₃)₃·6H₂O, 0.023 g Pt(NH₃)₄(NO3)₂, and 4.03 g Ca(NO₃)₂ were mixed together with 1696 g DI water in a container at room temperature, then heated to ~90 °C for 15 min. to reach a milk-like colloidal solution, to which 144.6 g aluminum isopropoxide and 53.7 g tetraethyl orthosilicate were slowly added. The mixture was stirred at 90 °C for at least 30 min., then an aqueous solution containing 17.97 g HNO₃ and 5.5 g DI water was added to the mixture. The final mixture was further heated at 86-90 °C with vigorous stirring for - 6 hours to provide a concentrated slurry/gel. The composition was dried in air at 120 °C for 16 hours and calcined at 600 °C in air for 4 hours.

Catalyst **A38** was prepared via sol-gel synthesis: 8.49 g Ga(NO₃)₃, 0.46 g KNO₃, 1.57 g Ce(NO₃)₃·6H₂O, 0.042 g Pt(NH₃)₄(NO3)₂, and 0.79 g Mn(NO₃)₂·6H₂O were mixed together with 1697 g DI water in a container at room temperature, then heated to ~90 °C for 15 min. to form a milk-like colloidal solution, to which 144.6 g aluminum isopropoxide and 50.7 g tetraethyl orthosilicate were slowly added. The mixture was stirred at 90 °C for 45 min., then an aqueous solution containing 18.0 g HNO₃ and 5.4 g DI water was added to the mixture. The final mixture was further heated at 86-90 °C while vigorously stirring for -5 hr to provide a concentrated slurry/gel. The as-made sample was dried in air at 120 °C for 16 hours and calcined at 600 °C in air for 4 hours.

Catalyst **A39** was prepared via sol-gel synthesis: 8.29 g Ga(NO₃)₃, 0.48 g KNO₃, 2.1 g Ce(NO₃)₃·6H₂O, 0.044 g Pt(NH₃)₄(NO3)₂, and 4.36 g Ca(NO₃)₂ were mixed together with 1696 g DI water in a container at room temperature, then heated to ~90 °C for 15 min. to form a milk-like colloidal solution, to which 144.6 g aluminum isopropoxide, 53.6 g tetraethyl orthosilicate, and 1.0 g zirconyl nitrate were slowly added. The mixture was stirred at 90 °C for 60 min., then an aqueous solution containing 18.0 g HNO₃ and 5.5 g DI water was added to the mixture. The final mixture was further heated at 86-90 °C while vigorously stirring for - 5 hr to provide a concentrated slurry/gel. The as-made sample was dried in air at 120 °C for 16 hours and calcined at 600 °C in air for 4 hours.

Catalyst **A40** was prepared via sol-gel synthesis: 8.3 g Ga(NO₃)₃, 0.46 g KNO₃, 1.6 g Ce(NO₃)₃·6H₂O, 0.044 g Pt(NH₃)₄(NO3)₂, and 4.1 g Ca(NO₃)₂ were mixed together with 1696 g DI water in a container at room temperature, then heated to ~90 °C for 15 min. to form a milk-like colloidal solution, to which 144.6 g aluminum isopropoxide, 53.8 g tetraethyl orthosilicate, and 0.7 g La(NO3)3·6H2O were slowly added. The mixture was stirred at 90 °C for 50 min., then an aqueous solution containing 18.1 g HNO₃ and 5.5 g DI water was added to the mixture. The final mixture was further heated at 86-90 °C while vigorously stirring for ~5 hr to provide a concentrated slurry/gel. The as-made sample was dried in air at 120 °C for 16 hours and calcined at 600 °C in air for 4 hours.

Catalyst **A41** was prepared via sol-gel synthesis: 8.3 g Ga(NO₃)₃, 0.48 g KNO₃, 1.6 g Ce(NO₃)₃·6H₂O, 0.043 g Pt(NH₃)₄(NO3)₂, and 1.22 g Fe(NO₃)₃·9H₂O were mixed together with 1696 g DI water in a container at room temperature, then heated to ~90 °C for 15 min. to form a milk-like colloidal solution, to which 144.7 g aluminum isopropoxide and 50.6 g tetraethyl orthosilicate were slowly added. The mixture was stirred at 90 °C for 50 min., then an aqueous solution containing 18.1 g HNO₃ and 5.5 g DI water was added to the mixture. The final mixture was further heated at 86-90 °C while vigorously stirring for ~5 hr to provide a concentrated slurry/gel. The as-made sample was dried in air at 120 °C for 16 hours and calcined at 600 °C in air for 4 hours.

Catalyst **A42** was prepared via sol-gel synthesis: 8.3 g Ga(NO₃)₃, 0.32 g KNO₃, 1.6 g Ce(NO₃)₃·6H₂O, 0.040 g Pt(NH₃)₄(NO3)₂, and 0.044 g SnCl₄·4H₂O, 3.6 g Mg(NO₃)₂·6H₂O were mixed together with 1696 g DI water in a container at room temperature, then heated to ~90 °C for 15 min. to form a milk-like colloidal solution, to which 106.3 g aluminum isopropoxide and 86.9 g tetraethyl orthosilicate were slowly added. The mixture was stirred at 90 °C for 70 min., then an aqueous solution containing 17.9 g HNO₃ and 5.5 g DI water was added to the mixture. The final mixture was further heated at 86-90 °C while vigorously stirring for ~5 hr to provide a concentrated slurry/gel. The as-made sample was dried in air at 120 °C for 16 hours and calcined at 600 °C in air for 4 hours.

Catalyst **A43** was prepared via sol-gel synthesis: 12.9 g Ga(NO₃)₃, 0.46 g KNO₃, 1.6 g Ce(NO₃)₃·6H₂O, 0.043 g Pt(NH₃)₄(NO3)₂, and 4.2 g Ca(NO₃)₂ were mixed together with 1696 g DI water in a container at room temperature, then heated to ~90 °C for 15 min. to form a milk-like colloidal solution, to which 124.7 g aluminum isopropoxide, 68.7 g tetraethyl orthosilicate, and 1.0 g zirconyl nitrate were slowly added. The mixture was stirred at 90 °C for 40 min., then an aqueous solution containing 18.0 g HNO₃ and 5.6 g DI water was added to the mixture. The final mixture was further heated at 86-90 °C while vigorously stirring for ~5 hr to provide a concentrated slurry/gel. The as-made sample was dried in air at 120 °C for 16 hours and calcined at 600 °C in air for 4 hours.

Catalyst **A44** was prepared via sol-gel synthesis: 12.9 g Ga(NO₃)₃, 0.46 g KNO₃, 1.6 g Ce(NO₃)₃·6H₂O, 0.042 g Pt(NH₃)₄(NO3)₂, and 0.72 g Mg(NO₃)₂·4H₂O were mixed together with 1696 g DI water in a container at room temperature, then heated to ~90 °C for 15 min. to form a milk-like colloidal solution, to which 124.1 g aluminum isopropoxide, 68.7 g tetraethyl orthosilicate, and 1.0 g zirconyl nitrate were slowly added. The mixture was stirred at 90 °C for 60 min., then an aqueous solution containing 18.1 g HNO₃ and 5.5 g DI water was added to the mixture. The final mixture was further heated at 86-90 °C while vigorously stirring for ~ 6 hr to provide a concentrated slurry/gel. The as-made sample was dried in air at 120 °C for 16 hours and calcined at 600 °C in air for 4 hours.

Catalyst **A45** was prepared via sol-gel synthesis: 8.3 g Ga(NO₃)₃, 0.46 g KNO₃, 1.6 g Ce(NO₃)₃·6H₂O, 0.041 g Pt(NH₃)₄(NO3)₂, and 1.94 g Ba(NO₃)₂ were mixed together with 1696 g DI water in a container at room temperature, then heated to ~90 °C for 15 min. to form a milk-like colloidal solution, to which 144.6 g aluminum isopropoxide, 55.6 g tetraethyl orthosilicate, and 0.4 g La(NO3)3·6H2O were slowly added. The mixture was stirred at 90 °C for ~60 min., then an aqueous solution containing 18.0 g HNO₃ and 5.5 g DI water was added to the mixture. The final mixture was further heated at 86-90 °C while vigorously stirring for ~ 6 hr to provide a concentrated slurry/gel. The as-made sample was dried in air at 120 °C for 16 hours and calcined at 600 °C in air for 4 hours.

Catalyst **A46** was prepared via sol-gel synthesis: 8.5 g Ga(NO₃)₃, 0.44 g KNO₃, 1.6 g Ce(NO₃)₃·6H₂O, 0.043 g Pt(NH₃)₄(NO3)₂, and 4.0 g Ca(NO₃)₂ were mixed together with 1696 g DI water in a container at room temperature, then heated to ~90 °C for 15 min. to form a milk-like colloidal solution, to which 144.6 g aluminum isopropoxide, 55.6 g tetraethyl orthosilicate, and 0.4 g La(NO3)3·6H2O were slowly added. The mixture was stirred at 90 °C for ~50 min., then an aqueous solution containing 18.0 g HNO₃ and 5.5 g DI water was added to the mixture. The final mixture was further heated at 86-90 °C while vigorously stirring for ~ 6 hr to provide a concentrated slurry/gel. The as-made sample was dried in air at 120 °C for 16 hours and calcined at 600 °C in air for 4 hours.

Catalyst **A47** was prepared via sol-gel synthesis: 8.3 g Ga(NO₃)₃, 0.47 g KNO₃, 1.6 g Ce(NO₃)₃·6H₂O, 0.041 g Pt(NH₃)₄(NO3)₂, and 2.5 g Sr(NO₃)₂ were mixed together with 1696 g DI water in a container at room temperature, then heated to ~90 °C for 15 min. to form a milk-like colloidal solution, to which 144.6 g aluminum isopropoxide, 55.7 g tetraethyl orthosilicate, and 0.4 g La(NO3)3·6H2O were slowly added. The mixture was stirred at 90 °C for ~50 min., then an aqueous solution containing 18.0 g HNO₃ and 5.5 g DI water was added to the mixture. The final mixture was further heated at 86-90 °C while vigorously stirring for ~ 5 hr to provide a concentrated slurry/gel. The as-made sample was dried in air at 120 °C for 16 hours and calcined at 600 °C in air for 4 hours.

Catalyst **A48** was prepared via sol-gel synthesis: 8.3 g Ga(NO₃)₃, 0.46 g KNO₃, 1.6 g Ce(NO₃)₃·6H₂O, 0.023 g Pt(NH₃)₄(NO3)₂, and 4.0 g Ca(NO₃)₂ were mixed together with 1696 g DI water in a container at room temperature, then heated to ~90 °C for 15 min. to form a milk-like colloidal solution, to which 146.4 g aluminum isopropoxide and 38.5 g NALCO^{®} 2327 were slowly added. The mixture was stirred at 90 °C for ~60 min., then an aqueous solution containing 18.1 g HNO₃ and 5.5 g DI water was added to the mixture. The final mixture was further heated at 86-90 °C while vigorously stirring for ~ 5 hr to provide a concentrated slurry/gel. The as-made sample was dried in air at 120 °C for 16 hours and calcined at 600 °C in air for 4 hours.

Catalyst **A49** was prepared via sol-gel synthesis: 8.3 g Ga(NO₃)₃, 0.46 g KNO₃, 1.6 g Ce(NO₃)₃·6H₂O, 0.023 g Pt(NH₃)₄(NO3)₂, and 4.0 g Ca(NO₃)₂ were mixed together with 1696 g DI water in a container at room temperature, then heated to ~90 °C for 15 min. to form a milk-like colloidal solution, to which 55.6 g bayerite and 53.6 g tetraethyl orthosilicate were slowly added. The mixture was stirred at 90 °C for ~60 min., then an aqueous solution containing 18.0 g HNO₃ and 5.5 g DI water was added to the mixture. The final mixture was further heated at 86-90 °C while vigorously stirring for ~ 5 hr to provide a concentrated slurry/gel. The as-made sample was dried in air at 120 °C for 16 hours and calcined at 600 °C in air for 4 hours.

Catalyst **A50** was prepared via sol-gel synthesis: 8.75 g Ga(NO₃)₃, 0.49 g KNO₃, 6.6 g Ce(NO₃)₃·6H₂O, 0.027 g Pt(NH₃)₄(NO3)₂, and 4.3 g Ca(NO₃)₂ were mixed together with 1696 g DI water in a container at room temperature, then heated to ~90 °C for 15 min. to form a milk-like colloidal solution, to which 144.6 aluminum isopropoxide and 53.6 g tetraethyl orthosilicate and 1.4 g zirconium n-propoxide were slowly added. The mixture was stirred at 90 °C for ~60 min., then an aqueous solution containing 18.0 g HNO₃ and 5.5 g DI water was added to the mixture. The final mixture was further heated at 86-90 °C while vigorously stirring for ~ 5.5 hr to provide a concentrated slurry/gel. The as-made sample was dried in air at 120 °C for 16 hours and calcined at 600 °C in air for 4 hours.

Comparative, alumina-supported gallium catalysts **C1** and **C2,** lacking cerium and/or promoters, were prepared according to conventional methods.

Comparative catalyst **C3** was prepared via sol-gel synthesis: 1.34 g Ga(NO₃)₃, 0.08 g KNO₃, and 0.38 g Ce(NO₃)₃·6H₂O were mixed together with 424 g DI water in a container at room temperature, then heated to 90 °C for at 15 min. to reach a homogenous solution, to which 48.1 g aluminum isopropoxide was slowly added. The mixture was stirred at 90 °C for at least 30 min., then an aqueous solution containing 4.5 g HNO₃ and 1.4 g DI water was added to the mixture. The final mixture was further heated at 86-90 °C with vigorous stirring for ~ 3 hours. The composition was dried in air at 120 °C for 16 hours and calcined at 600 °C in air for 4 hours.

**Table 1. Catalyst Compositions**

| | | | M1 | | M1-B | | M2 | |
|---|---|---|---|---|---|---|---|---|
| Cat | Ga (wt.%) | Ce (wt. %) | material | wt.% | material | wt.% | material | wt.% |
| C1 | 3 | | | | | | K | 0.25 |
| C2 | 3 | | Pt | 0.1 | | | K | 0.25 |
| C3 | 3 | 1 | | | | | K | 0.25 |
| A1 | 3 | 1 | La | 0.1 | | | K | 0.25 |
| A2 | 3 | 1 | Pt | 0.1 | | | K | 0.25 |
| A3 | 3 | 1 | Pt | 0.1 | Sn | 0.05 | K | 0.25 |
| A4 | 6 | 1 | Pt | 0.1 | Sn | 0.05 | K | 0.25 |
| A5 | 3 | 1 | Ir | 0.1 | Sn | 0.05 | K | 0.25 |
| A6 | 6 | 1 | Ir | 0.1 | Sn | 0.05 | K | 0.25 |
| A7 | 6 | 1 | Ir | 0.1 | Sn | 0.05 | K | 0.25 |
| A8 | 12 | 1 | Ir | 0.1 | Sn | 0.05 | K | 0.25 |
| A9 | 4.5 | 1 | Ir | 0.2 | | | K | 0.25 |
| A10 | 6 | 1 | Ir | 0.1 | Sn | 0.05 | K | 0.35 |
| A11 | 6 | 1 | Ir | 0.1 | Sn | 0.05 | K | 0.25 |
| A12 | 4.5 | 1 | Ir | 0.2 | | | K | 0.25 |
| A13 | 4.5 | 1 | Ir | 0.2 | | | K | 0.25 |
| A14 | 4.5 | 1 | Ir | 0.2 | | | K | 0.25 |
| A15 | 4.5 | 1 | Pt | 0.1 | | | K | 0.25 |
| A16 | 4.5 | 1 | Pt | 0.1 | | | K | 0.25 |
| A17 | 12 | 1 | Pt | 0.1 | Sn | 0.05 | K | 0.25 |
| A18 | 4.5 | 1 | Pt | 0.1 | | | K | 0.35 |
| A19 | 4.5 | 1 | Pt | 0.1 | | | K | 0.35 |
| A20 | 4.5 | 1 | Pt | 0.1 | | | K | 0.35 |
| A21 | 12 | 1 | Ir | 0.1 | Sn | 0.05 | K | 0.35 |
| A22 | 4.5 | 9.4 | Ir | 0.1 | | | K | 0.25 |
| A23 | 4.5 | 1 | Pt | 0.1 | | | K | 0.35 |
| A24 | 4.5 | 1 | Pt | 0.1 | | | K | 0.25 |
| A25 | 4.5 | 1 | Pt | 0.1 | | | K | 0.35 |
| A26 | 9 | 1 | Pt | 0.1 | | | K | 0.35 |
| A27 | 4.5 | 1 | Pt | 0.1 | | | K | 0.35 |
| A28 | 4.5 | 1 | Pt | 0.1 | | | K | 0.25 |
| A29 | 4.5 | 1 | Pt | 0.1 | | | K | 0.25 |
| A30 | 4.5 | 1 | Pt | 0.1 | | | K | 0.25 |
| A31 | 4.5 | 1 | Pt | 0.1 | | | K | 0.25 |
| A32 | 4.5 | 1 | Pt | 0.1 | | | K | 0.25 |
| A33 | 4.5 | 1 | Pt | 0.1 | | | K | 0.25 |
| A34 | 4.5 | 1 | Pt | 0.1 | | | K | 0.25 |
| A35 | 4.5 | 1 | Pt | 0.1 | | | K | 0.25 |
| A36 | 4.5 | 1 | Pt | 0.1 | | | K | 0.35 |
| A37 | 4.5 | 1 | Pt | 0.025 | | | K | 0.35 |
| A38 | 4.5 | 1 | Pt | 0.05 | | | K | 0.35 |
| A39 | 4.5 | 1 | Pt | 0.05 | | | K | 0.35 |
| A40 | 4.5 | 1 | Pt | 0.05 | | | K | 0.35 |
| | | | La | 0.1 | | | | |
| A41 | 4.5 | 1 | Pt | 0.05 | | | K | 0.35 |
| A42 | 4.5 | 1 | Pt | 0.05 | | 0.05 | K | 0.25 |
| A43 | 7 | 1 | Pt | 0.05 | | | K | 0.35 |
| A44 | 7 | 1 | Pt | 0.05 | | | K | 0.35 |
| A45 | 4.5 | 1 | Pt | 0.05 | | | K | 0.35 |
| | | | La | 0.1 | | | | |
| A46 | 4.5 | 1 | Pt | 0.05 | | | K | 0.35 |
| | | | La | 0.1 | | | | |
| A47 | 4.5 | 1 | Pt | 0.05 | | | K | 0.35 |
| | | | La | 0.1 | | | | |
| A48 | 4.5 | 1 | Pt | 0.025 | | | K | 0.35 |
| A49 | 4.5 | 1 | Pt | 0.025 | | | K | 0.35 |
| A50 | 4.5 | 4 | Pt | 0.025 | | | K | 0.35 |

**Table 2. Catalyst Compositions**

| | M3 | | S1 | | | |
|---|---|---|---|---|---|---|
| Cat | material | wt.% | Al₂O₃ (wt.%) | SiO₂ (wt.%) | ZrO₂ (wt.%) | TiO₂ (wt.%) |
| C1 | | | | | | |
| C2 | | | | | | |
| C3 | | | 95.60 | | | |
| A1 | | | 95.6 | | | |
| A2 | | | 95.65 | | | |
| A3 | | | 95.65 | | | |
| A4 | | | 92.6 | | | |
| A5 | | | 95.6 | | | |
| A6 | | | 92.6 | | | |
| A7 | | | 69.45 | 23.15 | | |
| A8 | | | 86.6 | | | |
| A9 | Mg | 1 | 93.05 | | | |
| A10 | | | 92.5 | | | |
| A11 | Mg | 1 | 91.6 | | | |
| A12 | Mg | 1 | 93.05 | | | |
| A13 | Mg | 1.5 | 92.55 | | | |
| A14 | Ca | 2 | 91.05 | | | |
| A15 | Mg | 0.25 | 93.90 | | | |
| A16 | Mg | 0.3 | 70.39 | 23.46 | | |
| A17 | Mg | 1 | 64.2 | | 21.4 | |
| A18 | Mg | 0.3 | 70.31 | 23.44 | | |
| A19 | Mn | 0.3 | 70.31 | 23.44 | | |
| A20 | Ca | 3 | 68.29 | 22.76 | | |
| A21 | Mg | 0.3 | 81.89 | 4.31 | | |
| A22 | Mg | 1 | 84.75 | | 9.4 | |
| A23 | Mg | 1 | 83.75 | | 9.3 | |
| A24 | Mg | 0.6 | 70.16 | 23.39 | | |
| A25 | Mg | 1 | 83.75 | | | 9.31 |
| A26 | Mg | 1 | 79.7 | | 8.86 | |
| A27 | Ca | 2 | 69.04 | 23.01 | | |
| A28 | Mg | 0.3 | 79.77 | 14.08 | | |
| A29 | Mg | 0.3 | 61 | 32.85 | | |
| A30 | Mg | 0.3 | 84.47 | | 9.39 | |
| A31 | Mg | 0.3 | 51.62 | 42.23 | | |
| A32 | Mg | 0.3 | 75.08 | | 18.77 | |
| A33 | Mg | 0.3 | 65.7 | | 28.16 | |
| A34 | Mg | 0.3 | 84.47 | | | 9.39 |
| A35 | Mg | 0.3 | 75.08 | | | 18.77 |
| A36 | Mg | 0.3 | 70.36 | 23.46 | | |
| A37 | Ca | 2 | 69.08 | 23.03 | | |
| A38 | Mn | 0.3 | 70.35 | 23.45 | | |
| A39 | Ca | 2 | 68.33 | 22.78 | 0.9 | |
| A40 | Ca | 2 | 67.1 | 22.6 | | |
| A41 | Fe | 0.5 | 70.2 | 23.4 | | |
| A42 | Mg | 0.6 | 51.45 | 42.1 | | |
| A43 | Ca | 2 | 57.66 | 31.05 | 0.9 | |
| A44 | Mn | 0.3 | 58.75 | 31.64 | 0.9 | |
| A45 | Ba | 2 | 64.5 | 27.4 | | |
| A46 | Ca | 2 | 56.2 | 35.5 | | |
| A47 | Sr | 2 | 23.4 | 27.4 | | |
| A48 | Ca | 2 | 69.1 | 23 | | |
| A49 | Ca | 2 | 69.1 | 23 | | |
| A50 | Ca | 2 | 66.1 | 22.0 | 1.0 | |

### Example 2. Propane Dehydrogenation

Catalyst compositions prepared according to Example 1 were tested as prepared in a fixed-bed reactor. A feed containing 100 mol.% propane was passed over a 15 ml catalyst bed at a total pressure of 0.5 bar, at 2.0 h⁻¹ liquid hourly space velocity (LHSV), at a temperature within the range of 540-600 °C. The product effluent concentration at the reactor outlet was monitored with an in-line gas chromatograph (GC). Results are provided in Table 3, below.

**Table 3. Propane Dehydrogenation**

| Cat | T (°C) | Propane Conversion (%) | Propylene Selectivity (%) |
|---|---|---|---|
| C1 | 540 | 11.0 | 87.8 |
| C2 | 540 | 8.7 | 91.9 |
| C3 | 540 | 15.7 | 88.3 |
| A1 | 540 | 16.8 | 87.0 |
| A2 | 540 | 24.2 | 89.4 |
| A3 | 540 | 26.7 | 85.6 |
| A4 | 540 | 31.0 | 81.6 |
| A5 | 540 | 27.0 | 85.2 |
| A6 | 540 | 31.1 | 81.0 |
| A7 | 540 | 30.8 | 80.2 |
| A8 | 540 | 32.1 | 74.2 |
| A9 | 540 | 28.3 | 89.2 |
| A10 | 540 | 31.9 | 82.6 |
| A11 | 540 | 31.4 | 85.5 |
| A12 | 540 | 31.6 | 86.5 |
| A13 | 540 | 28.6 | 89.2 |
| A14 | 540 | 29.1 | 87.4 |
| A15 | 540 | 30.1 | 85.6 |
| A16 | 540 | 33.7 | 82.4 |
| A17 | 540 | 33.1 | 86.3 |
| A18 | 540 | 30.7 | 88.8 |
| A19 | 540 | 33.7 | 88.5 |
| A20 | 540 | 26.9 | 91.5 |
| A21 | 540 | 29.1 | 83.0 |
| A22 | 540 | 27.3 | 85.8 |
| A23 | 540 | 25.3 | 88.9 |
| A24 | 540 | 33.1 | 86.3 |
| A25 | 540 | 27.2 | 89.0 |
| A26 | 540 | 28.0 | 89.5 |
| A27 | 540 | 34.9 | 90.2 |
| A28 | 540 | 32.4 | 87.8 |
| A29 | 540 | 34.9 | 87.1 |
| A30 | 540 | 26.8 | 89.3 |
| A31 | 540 | 36.2 | 86.4 |
| A32 | 540 | 25.7 | 86.9 |
| A33 | 540 | 28.2 | 86.4 |
| A34 | 540 | 24.7 | 84.7 |
| A35 | 540 | 26.2 | 80.7 |
| A36 | 540 | 36.0 | 91.1 |
| A37 | 540 | 33.8 | 91.1 |
| A38 | 540 | 34.2 | 88.3 |
| A39 | 540 | 34.8 | 89.3 |
| A40 | 540 | 34.7 | 87.6 |
| A41 | 540 | 35.8 | 83.1 |
| A42 | 540 | 33.9 | 87.9 |
| A43 | 540 | 35.4 | 88.8 |
| A44 | 540 | 34.3 | 83.8 |
| A45 | 540 | 35.3 | 89.1 |
| A46 | 540 | 34.8 | 90.1 |
| A47 | 540 | 34.1 | 89.0 |
| A48 | 540 | 18.8 | 84.9 |
| A49 | 540 | 23.7 | 85.2 |
| A50 | 540 | 34.7 | 91.7 |

The results, shown in Table 3, demonstrate that the PtCe- and IrCe- promoted Ga catalysts provide hydrocarbon dehydrogenation efficiency better than conventional catalysts.

### Example 3. Promoter Comparison

Catalysts **C3** and **A1-A2,** catalysts prepared in a manner similar to that of Example 1 (catalyst **A51** and comparative catalysts **C4-C12,** shown in Table 4, below), and a commercially available CrOx/Al₂O₃ catalyst (**Comm**.) were tested in a fixed-bed reactor in a manner similar to that of Example 2.

**Table 4. Catalyst Compositions**

| | | | M1 | | M2 | | S1 |
|---|---|---|---|---|---|---|---|
| Cat | Ga (wt.%) | Ce (wt. %) | material | wt.% | material | wt.% | Al₂O₃ (wt.%) |
| A51 | 3 | 1 | Ir | 0.1 | K | 0.25 | 95.65 |
| C4 | 3 | 1 | Ru | 0.1 | K | 0.25 | 95.65 |
| C5 | 3 | 1 | Pd | 0.1 | K | 0.25 | 95.65 |
| C6 | 3 | 1 | Co | 0.1 | K | 0.25 | 95.65 |
| C7 | 3 | 1 | Fe | 0.1 | K | 0.25 | 95.65 |
| C8 | 3 | 1 | Ni | 0.1 | K | 0.25 | 95.65 |
| C9 | 3 | 1 | In | 0.1 | K | 0.25 | 95.65 |
| C10 | 3 | 1 | Cu | 0.1 | K | 0.25 | 95.65 |
| C11 | 3 | 1 | Zn | 0.1 | K | 0.25 | 95.65 |
| C12 | 3 | 1 | Sn | 0.1 | K | 0.25 | 95.65 |

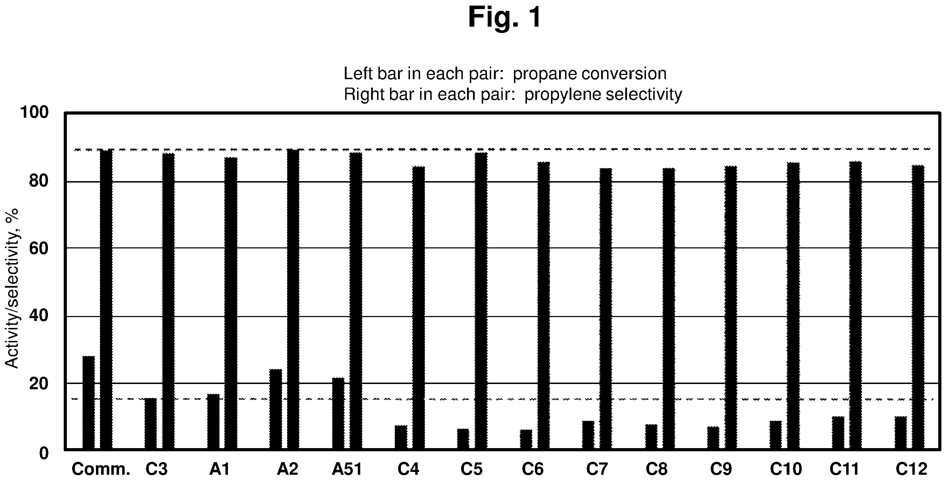

The results, shown in Figure 1, demonstrate that the promoted Ga- and Ce-containing catalysts provide hydrocarbon dehydrogenation efficiency comparable to or better than conventional catalysts.

### Example 4. 3-Cycle Activity/Selectivity Comparison

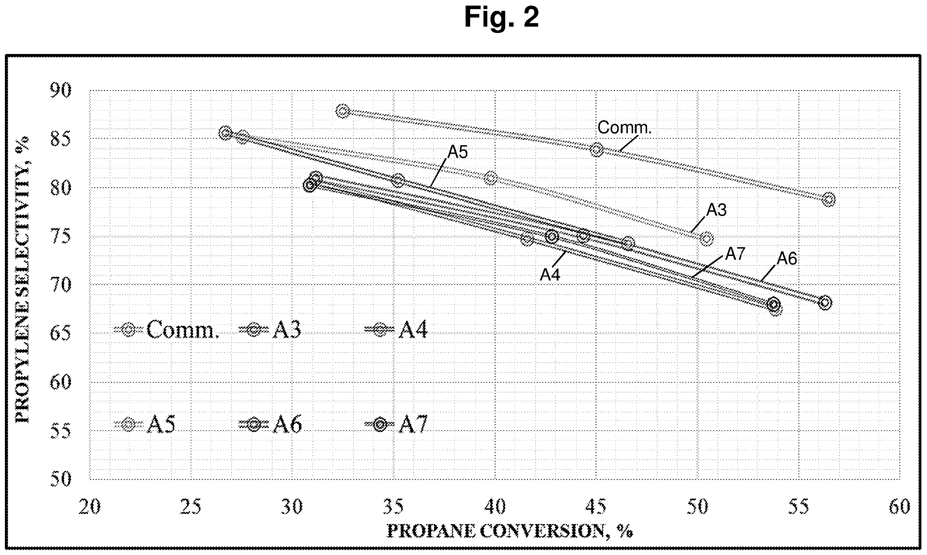

Catalysts **A3-A7,** a catalyst prepared in a manner similar to that of Example 1, and a commercially available CrOx/Al₂O₃ catalyst (**Comm**.) were tested in a fixed-bed reactor in a manner similar to that of Example 2, for three consecutive cycles. Activity and selectivity results for each cycle are provided in Figure 2.

The results, shown in Figure 2, demonstrate a negative correlation between conversion and selectivity. At a propane conversion of 45%, catalyst **A5** showed the best selectivity of the compositions tested, which was about 6% lower than that of the commercially available CrOx/Al₂O₃ catalyst.

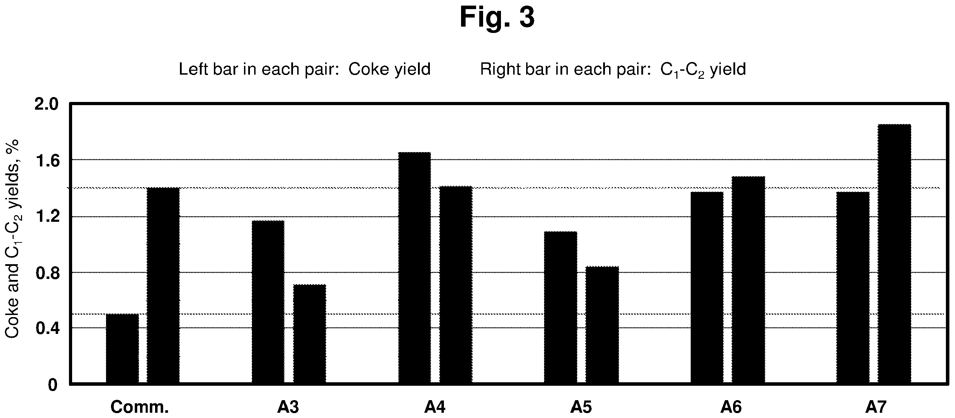

The coke and C₁-C₂ byproduct yields of the third cycle are provided in Figure 3. The catalyst compositions showed a level of coke production at least twice that of the commercial catalyst. Without intending to be bound by theory, the present inventors believe that degree of coke formation is due to higher surface acidity, which acidity would also explain the difference in selectivity noted above. Catalyst **A7** showed the highest C₁-C₂ byproduct yield. Without intending to be bound by theory, the present inventors believe that the byproduct yield increase relative to catalyst **A6** can be attributed, in part, to the inclusion of silica.

### Example 5. Performance Testing

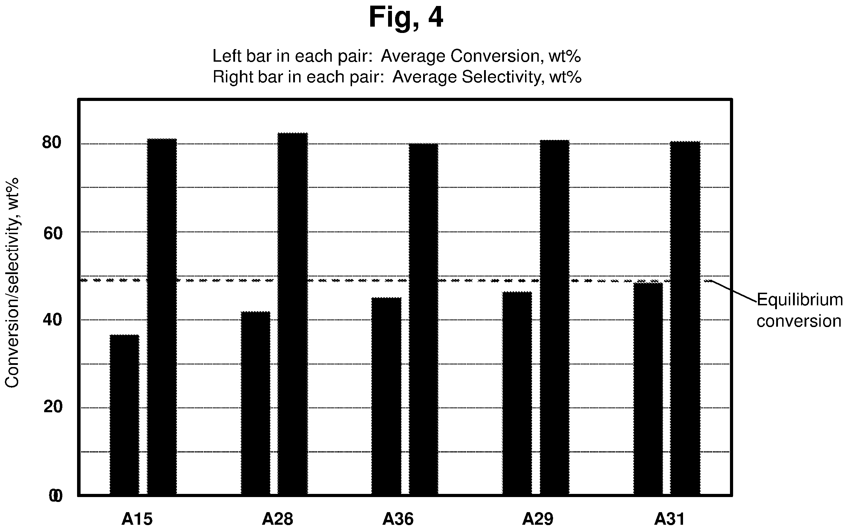

To evaluate the effects of incorporation of silica into the catalyst compositions, catalysts **A15, A28, A29, A31,** and **A36** were tested in a fixed-bed reactor in a manner similar to that of Example 2. Activity and selectivity results are provided in Figure 4.

The results, provided in Figure 4, show that propane conversion activity increased as the amount of silica included in the catalysts increased. The conversion activity of catalyst **A31** was 48.2%, very close to the calculated thermodynamic equilibrium conversion (48.9%). Selectivity changed negligibly.

### Example 6. Performance Testing

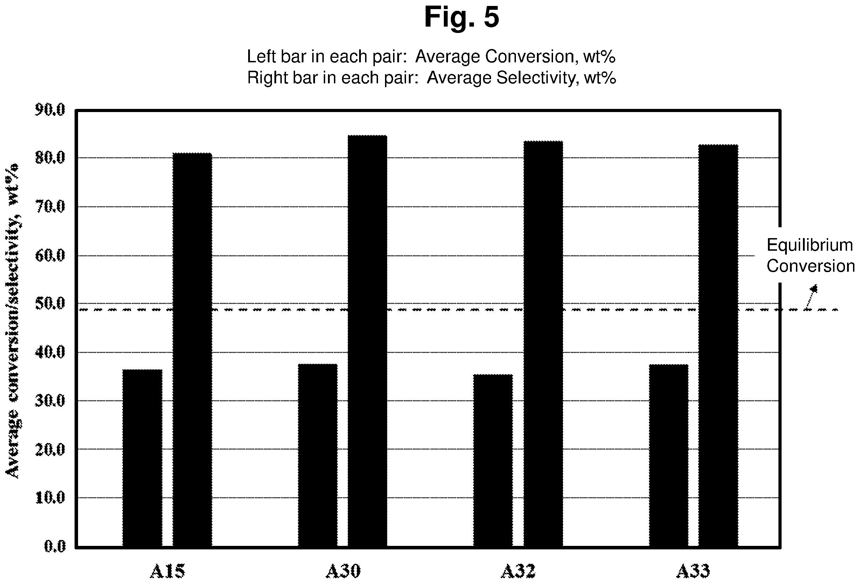

To evaluate the effects of incorporation of zirconia into the catalyst compositions, catalysts **A15, A30, A32,** and **A33** were tested in a fixed-bed reactor in a manner similar to that of Example 2. Activity and selectivity results are provided in Figure 5.

The results show that propane conversion activity and selectivity changed negligibly as the amount of zirconia included in the catalysts increased.

### Example 7. Performance Testing

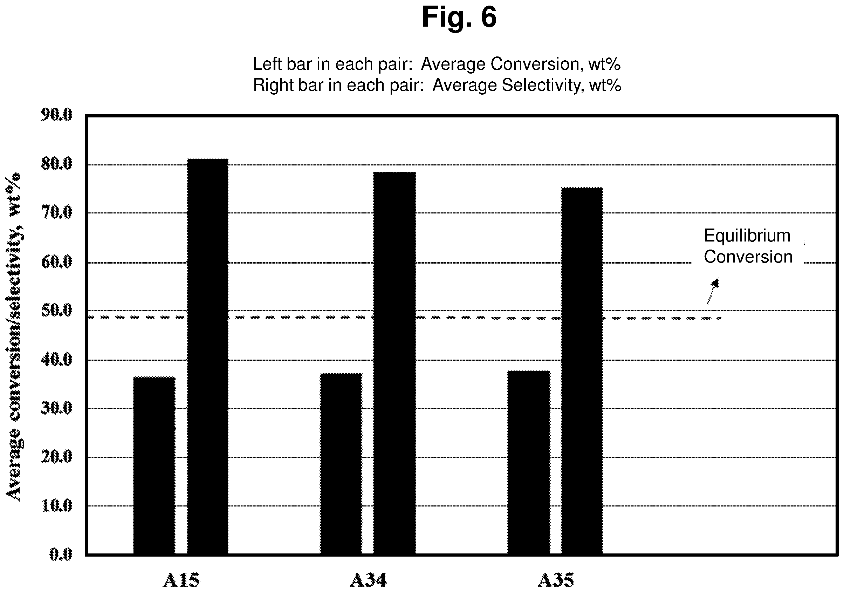

To evaluate the effects of incorporation of titania into the catalyst compositions, catalysts **A15, A34, and A35** were tested in a fixed-bed reactor in a manner similar to that of Example 2. Activity and selectivity results are provided in Figure 6.

The results show that propane conversion activity changed negligibly as the amount of titania included in the catalysts increased. Selectivity decreased gradually as the amount of titania included increased.

### Example 8. Performance Testing

To evaluate the effects of incorporation of magnesium or calcium into the catalyst compositions, catalysts **A24, A19, A27,** and **A29,** and a commercially available CrOx/Al₂O₃ catalyst (**Comm.**) were tested in a fixed-bed reactor in a manner similar to that of Example 2. Activity and selectivity results are provided in Table 5, below.

**Table 5. Propane Dehydrogenation**

| Property | Comm. (wt.%) | A24 (wt.%) | A19 (wt.%) | A27 (wt.%) | A29 (wt.%) |
|---|---|---|---|---|---|
| 1000°F | | | | | |
| C1 to C2 | 1.42 | 0.51 | 0.61 | 0.48 | 0.77 |
| Propane Conversion | 32.74 | 32.88 | 33.66 | 34.94 | 34.93 |
| Propylene Selectivity | 87.15 | 89.05 | 88.55 | 90.16 | 87.11 |
| Propylene Yield | 28.54 | 29.23 | 29.80 | 31.47 | 30.43 |
| Coke Yield | 0.60 | 0.93 | 1.07 | 0.85 | 1.15 |
| 1050°F | | | | | |
| C1 to C2 | 3.06 | 1.29 | 1.63 | 1.46 | 2.17 |
| Propane Conversion | 45.78 | 43.51 | 43.58 | 46.58 | 46.33 |
| Propylene Selectivity | 83.66 | 86.02 | 83.26 | 86.89 | 81.91 |
| Propylene Yield | 38.33 | 37.48 | 36.28 | 40.47 | 37.97 |
| Coke Yield | 1.23 | 1.45 | 1.60 | 1.42 | 2.22 |
| 1100°F | | | | | |
| C1 to C2 | 6.39 | 3.70 | 4.72 | 3.88 | 5.37 |
| Propane Conversion | 58.41 | 55.12 | 56.44 | 57.86 | 57.48 |
| Propylene Selectivity | 76.67 | 78.61 | 79.05 | 80.13 | 73.34 |
| Propylene Yield | 44.75 | 43.27 | 44.61 | 46.39 | 42.13 |
| Coke Yield | 3.20 | 3.27 | 3.60 | 2.97 | 4.56 |

The results show that the performance of the catalysts tested was acceptable.

Additional aspects of the disclosure are provided by the enumerated embodiments below, which can be combined and permuted in any fashion that is not logically or technically inconsistent.

## Claims

1. A calcined dehydrogenation catalyst composition comprising
gallium oxide, present in the composition in an amount within the range of 0.1 wt.% to 30 wt.%, calculated as Ga₂O₃ on a calcined basis;
cerium oxide, present in the composition in an amount within the range of 0.1 wt.% to 15 wt.%, calculated as CeO₂ on a calcined basis;
a promoter M1 selected from platinum, iridium, lanthanum, or a mixture thereof, present in the composition in an amount within the range of 0.005 wt.% to 4 wt.%, calculated as oxide on a calcined basis;
a promoter M2 selected from the group 1 elements, present in the composition in an amount within the range of 0.05 wt.% to 3 wt.%, calculated as oxide on a calcined basis;
a promotor M3 selected from Ca, Sr, Ba, Fe, present in the composition in an amount within the range of 0.05 wt.% to 10 wt.%, calculated as oxide on a calcined basis, and
a support S1 selected from alumina, silica, zirconia, titania, or a mixture thereof, present in the composition in an amount within the range of 60 wt.% to 99 wt.%, calculated as oxide on a calcined basis.

2. The catalyst composition of claim 1, wherein gallium oxide is present in the composition in an amount within the range of 2 wt.% to 15 wt.%, calculated as Ga₂O₃ on a calcined basis.

3. The catalyst composition of claim 1 or claim 2, wherein M1 is Pt or Ir or La or a combination of two or more thereof, and wherein M1 is present in the composition in an amount within the range of 0.01 wt.% to 2 wt.%, calculated as oxide on a calcined basis.

4. The catalyst composition of any of claims 1-3, wherein M2 is selected from Li, Na, K, and Cs, and wherein M2 is present in the composition in an amount within the range of 0.05 wt.% to 2 wt.%, calculated as oxide on a calcined basis.

5. The catalyst composition of any of claims 1-4, further comprising a promoter M1-B selected from Sn and Pd, present in the composition in an amount up to 0.5 wt.%, optionally within the range of 0.005 wt.% to 0.5 wt.%, calculated as SnO₂ or PdO, respectively, on a calcined basis.

6. The catalyst composition of claim 1, wherein
gallium oxide is present in the composition in an amount within the range of 1 wt.% to 20 wt.%, or 2 wt.% to 15 wt.%, calculated as Ga₂O₃ on a calcined basis;
cerium oxide is present in the composition in an amount within the range of 0.1 wt.% to 10 wt.%, or 0.1 wt.% to 3 wt.%, calculated as CeO₂ on a calcined basis;
M1 is Pt, Ir, La, or a mixture thereof, present in the composition in an amount within the range of 0.01 wt.% to 2 wt.%, or 0.02 wt.% to 1 wt.%, calculated as oxide on a calcined basis; and
M2 is K, present in the composition in an amount within the range of 0.05 wt.% to 2 wt.%, or 0.1 wt.% to 1 wt.%, calculated as K₂O on a calcined basis

7. The catalyst composition of claim 6 further comprising M1-B, wherein M1-B is Sn or Pd, present in an amount within the range of 0.005 wt.% to 0.5 wt.%, calculated as SnO₂ or PdO, respectively, on a calcined basis.

8. The catalyst composition of claim 6 or claim 7, wherein M3 is present in an amount within the range of 0.05 wt.% to 7 wt.%, calculated as oxide on a calcined basis.

9. The catalyst composition of any of claims 1-8, wherein S1 is alumina, a mixture of alumina and silica, a mixture of alumina and zirconia, of a mixture of alumina and titania and is present in the composition in an amount within the range of 70 wt.% to 99 wt.%.

10. The catalyst composition of any of claims 1-9, wherein
S1 is a mixture of alumina, silica, and zirconia;
alumina and silica are present in a weight ratio within the range of 0.5:1 to 25:1; and
alumina and zirconia are present in a weight ratio within the range of 90:1 to 40:1.

11. The catalyst composition of any of claims 1-10, wherein the composition comprises none of or less than 1 wt.% of the lanthanides other than lanthanum and cerium, and comprises no more than 0.5 wt% chromium.

12. A method for preparing a dehydrogenation catalyst composition according to any of claims 1-11, comprising
providing an aqueous solution comprising
a gallium source;
a cerium source;
an M1 source;
an M2 source;
an M3 source; and
an S1 source;
forming a solid from the solution; and
calcining the composition so formed.

13. Use of a catalyst composition of according to any of the claims 1-11 for the dehydrogenation of a hydrocarbon.

## Patentansprüche

1. Eine kalzinierte Dehydrierungskatalysatorzusammensetzung, umfassend
Galliumoxid, das in der Zusammensetzung in einer Menge im Bereich von 0,1 Gew.- % bis 30 Gew.-% vorhanden ist, berechnet als Ga₂O₃ auf kalzinierter Basis;
Ceroxid, das in der Zusammensetzung in einer Menge im Bereich von 0,1 Gew.-% bis 15 Gew.-%, berechnet als CeO₂ auf kalzinierter Basis, vorliegt;
einen Promotor M1, ausgewählt aus Platin, iridium, Lanthan oder einer Mischung davon, der in der Zusammensetzung in einer Menge im Bereich von 0,005 Gew.- % bis 4 Gew.-%, berechnet als Oxid auf kalzinierter Basis, vorliegt;
einen Promotor M2, ausgewählt aus den Elementen der Gruppe 1, der in der Zusammensetzung in einer Menge im Bereich von 0,05 Gew.-% bis 3 Gew.-%, berechnet als Oxid auf kalzinierter Basis, vorliegt;
einen Promotor M3, ausgewählt aus Ca, Sr, Ba, Fe, der in der Zusammensetzung in einer Menge im Bereich von 0,05 Gew.-% bis 10 Gew.-%, berechnet als Oxid auf kalzinierter Basis, vorhanden ist, und
einen Träger S1, ausgewählt aus Aluminiumoxid, Siliciumdioxid, Zirkoniumdioxid, Titandioxid oder einer Mischung davon, der in der Zusammensetzung in einer Menge im Bereich von 60 Gew.-% bis 99 Gew.-%, berechnet als Oxid auf kalzinierter Basis, vorhanden ist.

2. Katalysatorzusammensetzung nach Anspruch 1, wobei Galliumoxid in der Zusammensetzung in einer Menge im Bereich von 2 Gew.-% bis 15 Gew.-%, berechnet als Ga₂O₃ auf einer kalzinierten Basis, vorhanden ist.

3. Katalysatorzusammensetzung nach Anspruch 1 oder Anspruch 2, wobei M1 Pt oder Ir oder La oder eine Kombination von zwei oder mehr davon ist und wobei M1 in der Zusammensetzung in einer Menge im Bereich von 0,01 Gew.-% bis 2 Gew.-%, berechnet als Oxid auf kalzinierter Basis, vorhanden ist.

4. Katalysatorzusammensetzung nach einem der Ansprüche 1 bis 3, wobei M2 aus Li, Na, K und Cs ausgewählt ist und wobei M2 in der Zusammensetzung in einer Menge im Bereich von 0,05 Gew.-% bis 2 Gew.-%, berechnet als Oxid auf kalzinierter Basis, vorhanden ist.

5. Katalysatorzusammensetzung nach einem der Ansprüche 1 bis 4, die ferner einen Promotor M1-B umfasst, der aus Sn und Pd ausgewählt ist und in der Zusammensetzung in einer Menge von bis zu 0,5 Gew.-%, gegebenenfalls im Bereich von 0,005 Gew.-% bis 0,5 Gew.-%, berechnet als SnO₂ bzw. PdO, auf kalzinierter Basis vorliegt.

6. Katalysatorzusammensetzung nach Anspruch 1, wobei
Galliumoxid in der Zusammensetzung in einer Menge im Bereich von 1 Gew.-% bis 20 Gew.-% oder 2 Gew.-% bis 15 Gew.-%, berechnet als Ga₂O₃ auf kalzinierter Basis, vorhanden ist;
Ceroxid in der Zusammensetzung in einer Menge im Bereich von 0,1 Gew.-% bis 10 Gew.-% oder 0,1 Gew.-% bis 3 Gew.-%, berechnet als CeO₂ auf kalzinierter Basis, vorhanden ist;
M1 Pt, Ir, La oder eine Mischung davon ist, die in der Zusammensetzung in einer Menge im Bereich von 0,01 Gew.-% bis 2 Gew.-% oder 0,02 Gew.-% bis 1 Gew.- %, berechnet als Oxid auf kalzinierter Basis, vorhanden ist; und
M2 ist K, das in der Zusammensetzung in einer Menge im Bereich von 0,05 Gew.-% bis 2 Gew.-% oder 0,1 Gew.-% bis 1 Gew.-%, berechnet als K₂ O auf kalzinierter Basis, vorliegt

7. Katalysatorzusammensetzung nach Anspruch 6, die ferner M1-B umfasst, wobei M1-B Sn oder Pd ist, das in einer Menge im Bereich von 0,005 Gew.-% bis 0,5 Gew.-%, berechnet als SnO₂ bzw. PdO, auf einer kalzinierten Basis vorliegt.

8. Katalysatorzusammensetzung nach Anspruch 6 oder 7, wobei M3 in einer Menge im Bereich von 0,05 Gew.-% bis 7 Gew.-%, berechnet als Oxid auf kalzinierter Basis, vorhanden ist.

9. Katalysatorzusammensetzung nach einem der Ansprüche 1 bis 8, wobei S1 Aluminiumoxid, ein Gemisch aus Aluminiumoxid und Siliciumdioxid, ein Gemisch aus Aluminiumoxid und Zirkoniumdioxid oder ein Gemisch aus Aluminiumoxid und Titandioxid ist und in der Zusammensetzung in einer Menge im Bereich von 70 Gew.-% bis 99 Gew.- % vorhanden ist.

10. Katalysatorzusammensetzung nach einem der Ansprüche 1-9, wobei
S1 ist eine Mischung aus Aluminiumoxid, Siliziumdioxid und Zirkoniumdioxid;
Aluminiumoxid und Siliziumdioxid in einem Gewichtsverhältnis im Bereich von 0,5:1 bis 25:1 vorhanden sind; und
Tonerde und Zirkoniumdioxid in einem Gewichtsverhältnis von 90:1 bis 40:1 vorhanden sind.

11. Katalysatorzusammensetzung nach einem der Ansprüche 1-10, wobei die Zusammensetzung keine oder weniger als 1 Gew.-% der Lanthaniden außer Lanthan und Cer und nicht mehr als 0,5 Gew.-% Chrom enthält.

12. Verfahren zur Herstellung einer Dehydrierungskatalysatorzusammensetzung nach einem der Ansprüche 1-11, umfassend
Bereitstellung einer wässrigen Lösung mit
eine Galliumquelle;
eine Ceriumquelle;
eine M1-Quelle;
eine M2-Quelle;
eine M3-Quelle; und
eine S1-Quelle;
Bildung eines Feststoffs aus der Lösung; und
Kalzinierung der so gebildeten Zusammensetzung.

13. Verwendung einer Katalysatorzusammensetzung nach einem der Ansprüche 1 bis 11 für die Dehydrierung eines Kohlenwasserstoffs.

## Revendications

1. Une composition de catalyseur de déshydrogénation calcinée comprenant
oxyde de gallium, présent dans la composition en une quantité comprise entre 0,1 % en poids et 30 % en poids, calculée en Ga₂O₃ sur une base calcinée;
oxyde de cérium, présent dans la composition en une quantité comprise entre 0,1 % en poids et 15 % en poids, calculée en CeO₂ sur une base calcinée;
un promoteur M1 choisi parmi le platine, l'iridium, le lanthane ou un mélange de ceux-ci, présent dans la composition en une quantité comprise entre 0,005 % en poids et 4 % en poids, calculée en tant qu'oxyde sur une base calcinée;
un promoteur M2 choisi parmi les éléments du groupe 1, présent dans la composition en une quantité comprise entre 0,05 % en poids et 3 % en poids, calculée en tant qu'oxyde sur une base calcinée ;
un promoteur M3 de choisi parmi Ca, Sr, Ba, Fe, présent dans la composition en une quantité comprise entre 0,05 % en poids et 10 % en poids, calculée en tant qu'oxyde sur une base calcinée, et
un support S1 choisi parmi l'alumine, la silice, la zircone, le dioxyde de titane ou un mélange de ceux-ci, présent dans la composition en une quantité comprise entre 60 % en poids et 99 % en poids, calculée en tant qu'oxyde sur une base calcinée.

2. Composition de catalyseur selon la revendication 1, dans laquelle l'oxyde de gallium est présent dans la composition en une quantité comprise entre 2 % en poids et 15 % en poids, calculée en tant que Ga₂O₃ sur une base calcinée.

3. Composition de catalyseur selon la revendication 1 ou la revendication 2, dans laquelle M1 est Pt ou Ir ou La ou une combinaison de deux ou plusieurs de ceux-ci, et dans laquelle M1 est présent dans la composition en une quantité dans la gamme de 0,01 % en poids à 2 % en poids, calculée en tant qu'oxyde sur une base calcinée.

4. Composition de catalyseur selon l'une quelconque des revendications 1 à 3, dans laquelle M2 est choisi parmi Li, Na, K et Cs, et dans laquelle M2 est présent dans la composition en une quantité comprise dans la gamme de 0,05 % en poids à 2 % en poids, calculée en tant qu'oxyde sur une base calcinée.

5. Composition de catalyseur de l'une quelconque des revendications 1 à 4, comprenant en outre un promoteur M1-B choisi parmi Sn et Pd, présent dans la composition en une quantité allant jusqu'à 0,5 % en poids, éventuellement dans la gamme de 0,005 % en poids à 0,5 % en poids, calculée en tant que SnO₂ ou PdO, respectivement, sur une base calcinée.

6. Composition de catalyseur selon la revendication 1, dans laquelle
l'oxyde de gallium est présent dans la composition en une quantité comprise entre 1 % en poids et 20 % en poids, ou entre 2 % en poids et 15 % en poids, calculée sous forme de Ga₂O₃ sur une base calcinée;
l'oxyde de cérium est présent dans la composition en une quantité comprise entre 0,1 % en poids et 10 % en poids, ou entre 0,1 % en poids et 3 % en poids, calculée en CeO₂ sur une base calcinée;
M1 est Pt, Ir, La, ou un mélange de ceux-ci, présent dans la composition en une quantité comprise dans la gamme de 0,01 % en poids à 2 % en poids, ou 0,02 % en poids à 1 % en poids, calculée en tant qu'oxyde sur une base calcinée; et
M2 est K, présent dans la composition en une quantité comprise entre 0,05 % en poids et 2 % en poids, ou entre 0,1 % en poids et 1 % en poids, calculée en tant que K₂O sur une base calcinée.

7. Composition de catalyseur de la revendication 6 comprenant en outre M1-B, dans laquelle M1-B est Sn ou Pd, présent en une quantité dans la gamme de 0,005 % en poids à 0,5 % en poids, calculée comme SnO₂ ou PdO, respectivement, sur une base calcinée.

8. Composition de catalyseur selon la revendication 6 ou la revendication 7, dans laquelle M3 est présent en une quantité comprise dans la gamme de 0,05 % en poids à 7 % en poids, calculée en tant qu'oxyde sur une base calcinée.

9. Composition de catalyseur de l'une quelconque des revendications 1 à 8, dans laquelle S1 est de l'alumine, un mélange d'alumine et de silice, un mélange d'alumine et de zircone, ou un mélange d'alumine et d'oxyde de titane, et est présent dans la composition en une quantité comprise entre 70 % en poids et 99 % en poids.

10. Composition de catalyseur de l'une quelconque des revendications 1 à 9, dans laquelle
S1 est un mélange d'alumine, de silice et de zircone;
l'alumine et la silice sont présentes dans un rapport pondéral compris entre 0,5:1 et 25:1 ; et
l'alumine et la zircone sont présentes dans un rapport pondéral compris entre 90:1 et 40:1.

11. Composition de catalyseur selon l'une quelconque des revendications 1 à 10, dans laquelle la composition ne comprend aucun ou moins de 1 % en poids des lanthanides autres que le lanthane et le cérium, et ne comprend pas plus de 0,5 % en poids de chrome.

12. Méthode de préparation d'une composition de catalyseur de déshydrogénation selon l'une quelconque des revendications 1 à 11comprenant la mise à disposition
d'une solution aqueuse comprenant
une source de gallium;
une source de cérium;
une source M1;
une source M2;
une source M3; et
une source S1;
former un solide à partir de la solution; et
calciner la composition ainsi formée.

13. Utilisation d'une composition catalytique selon l'une quelconque des revendications 1 à 11 pour la déshydrogénation d'un hydrocarbure.
